# EUROPEAN PATENT APPLICATION

(11) **EP 4 349 994 A1**
(43) Date of publication of application: **10.04.2024**
(21) Application number: 22828838.7
(22) Date of filing: 24.06.2022
(51) Int. Cl.: C12P 7/62, C12P 7/18, C12N 15/70, C12N 9/10, C12N 9/88

(54) **NOVEL METHOD FOR PRODUCING POLY-4-HYDROXYBUTYRATE AND 1,4-BUTANEDIOL**

(30) Priority: 25.06.2021 KR 20210083270
(71) Applicant: CJ Cheiljedang Corporation, Seoul 04560 (KR)
(72) Inventor: KIM, Seo Hyoung, Seoul 04560 (KR); LEE, Kyung-Chang, Seoul 04560 (KR); LIM, Jae Hyung, Seoul 04560 (KR); HA, Hyo-Seok, Seoul 04560 (KR); CHANG, Dong-Eun, Seoul 04560 (KR)
(74) Representative: Regimbeau
(86) International application number: PCT/KR2022/009082
(87) International publication number: WO 2022/270991

(57) **Abstract**

The present disclosure relates to a novel method of producing poly-4-hydroxybutyrate and/or 1,4-butanediol, and a microorganism using a poly-4-hydroxybutyrate production pathway.

## Description

### [Technical Field]

The present disclosure relates to a novel method of producing poly-4-hydroxybutyrate and/or 1,4-butanediol, and a microorganism using a poly-4-hydroxybutyrate production pathway.

### [Background Art]

In order to produce 1,4-butanediol, various studies are being conducted to develop a microorganism for highly efficient production and a fermentation process technology. Production of 1,4-butanediol using microorganisms is usually accompanied by a 4-hydroxybutylaldehyde production reaction (US 9121042 B2). However, 4-hydroxybutylaldehyde, which is an aldehyde, has a disadvantage in that it is harmful to microorganisms.

In addition, one of the technologies for 1,4-butanediol bio-production known thus far is a method of directly producing 1,4-butanediol from glucose in microorganisms through fermentation as direct fermentation of 1,4-butanediol. However, there are restrictions on productivity due to toxicity of 1,4-butanediol itself to microorganisms. Accordingly, there is still a need for research on effectively increasing the production capacity of poly-4-hydroxybutyrate and/or 1,4-butanediol.

### [Disclosure]

### [Technical Problem]

The problem of the present disclosure to be solved is to provide a novel method of producing poly-4-hydroxybutyrate and 1,4-butanediol and a microorganism for producing poly-4-hydroxybutyrate and/or 1,4-butanediol.

### [Technical Solution]

An object of the present disclosure is to provide a method of producing 1,4-butanediol.

Another object of the present disclosure is to provide a microorganism for producing poly-4-hydroxybutyrate and/or 1,4-butanediol.

Still another object of the present disclosure is to provide a method of producing poly-4-hydroxybutyrate.

### [Effect of the invention]

When a method or a microorganism of the present disclosure is used, effective production of poly-4-hydroxybutyrate and 1 ,4-butanediol is possible.

### [Brief Description of Drawings]

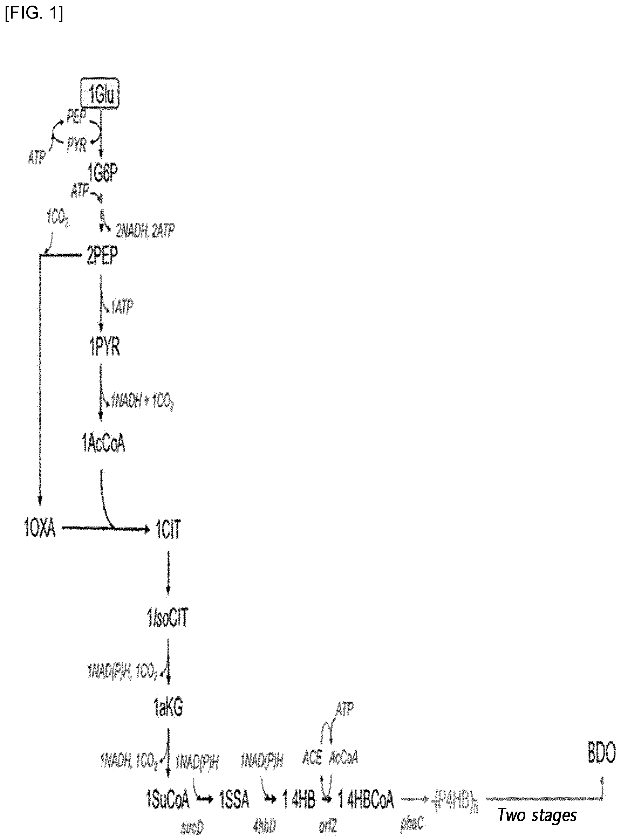

FIG. 1 illustrates a production pathway of poly-4-hydroxybutyrate and 1,4-butanediol using an oxidative TCA cycle;

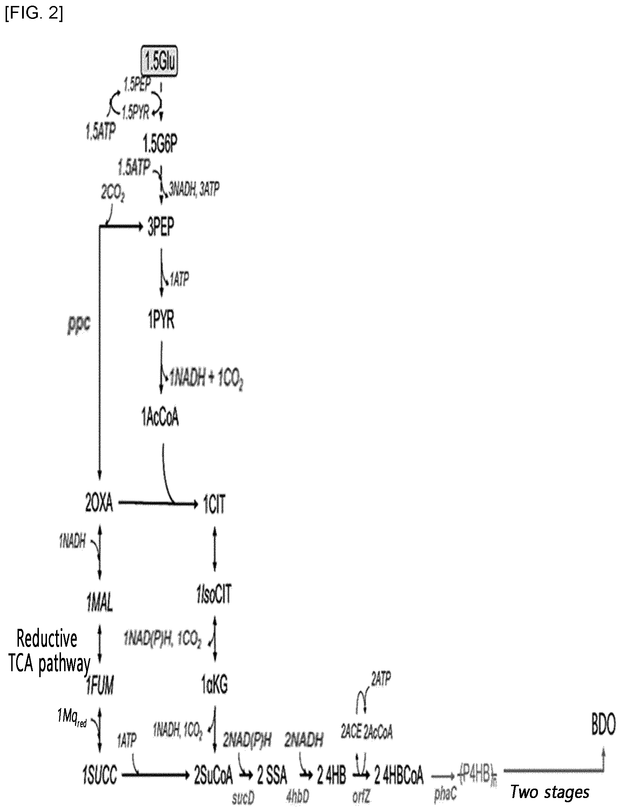

FIG. 2 illustrates a production pathway of poly-4-hydroxybutyrate and 1,4-butanediol using a reductive TCA cycle;

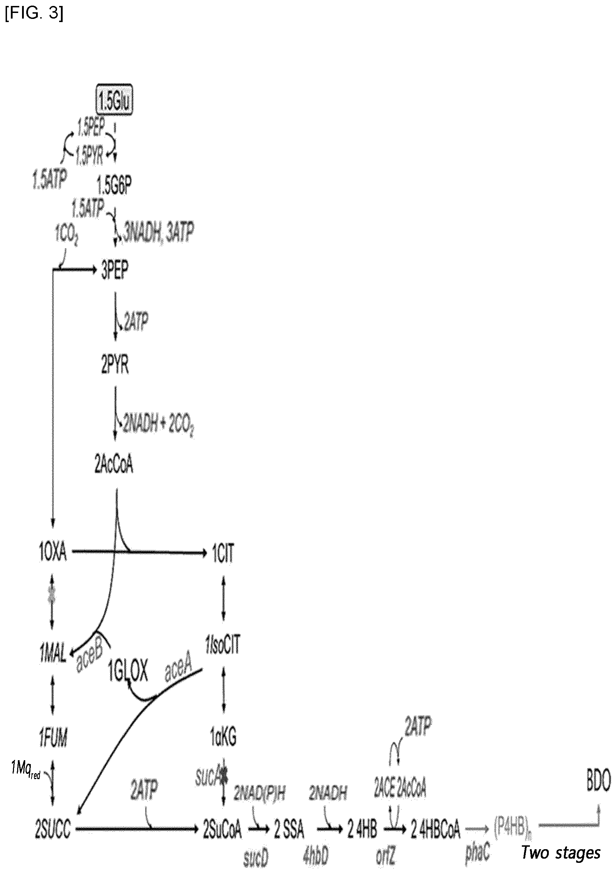

FIG. 3 illustrates a production pathway of poly-4-hydroxybutyrate and 1,4-butanediol using a glyoxylate cycle;

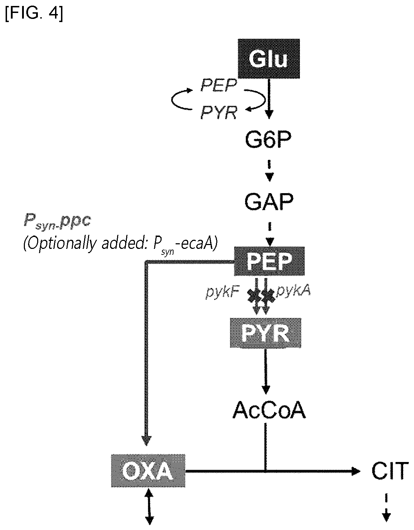

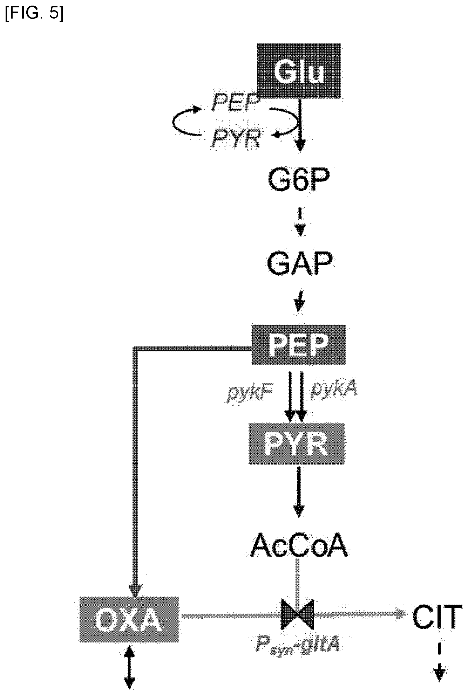

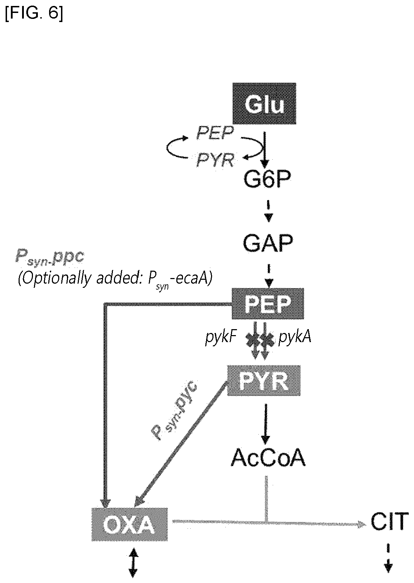

FIGS. 4 to 6 illustrate a method of enhancing a phosphoenolpyruvateoxaloacetate pathway in the production pathway of poly-4-hydroxybutyrate and 1,4-butanediol using the reductive TCA cycle;
FIG. 7 illustrates results of producing poly-4-hydroxybutyrate using the reductive TCA cycle; and
FIG. 8 illustrates results of producing poly-4-hydroxybutyrate using the glyoxylate cycle.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a method of producing 1,4-butanediol, the method including the following (1) to (5):
(1) converting succinyl-CoA (SuCoA) to succinate semialdehyde (SSA);
(2) converting succinate semialdehyde (SSA) to 4-hydroxybutyrate (4HB);
(3) converting 4-hydroxybutyrate (4HB) to 4-hydroxybutyryl CoA (4HBCoA);
(4) producing poly-4-hydroxybutyrate (P4HB) by polymerizing two or more of 4-hydroxybutyryl CoA (4HBCoA); and
(5) degrading poly-4-hydroxybutyrate to 1,4-butanediol.

(1) to (4) may use any one or more selected from the group consisting of any one or more polypeptides selected from the group consisting of succinate semialdehyde dehydrogenase, 4-hydroxybutyric acid dehydrogenase, 4-hydroxybutyryl-CoA transferase, and poly(3-hydroxyalkanoate) polymerase; a microorganism including the polypeptide, a polynucleotide encoding the polypeptide, a vector including the polynucleotide, or a combination thereof; and a culture thereof, respectively, but are not limited thereto.

Any one or more selected from the group consisting of succinate semialdehyde dehydrogenase, 4-hydroxybutyric acid dehydrogenase, 4-hydroxybutyryl-CoA transferase, and poly(3-hydroxyalkanoate) polymerase may be enhanced by a microorganism including a foreign polypeptide, a polynucleotide encoding the foreign polypeptide, or a vector including the polynucleotide, but are not limited thereto.

In direct production of 1,4-butanediol by glucose fermentation, there is a restriction on the productivity due to the toxicity of 1,4-butanediol to microorganisms. In contrast, in the present disclosure, cell-friendly poly-4-hydroxybutyrate is preferentially produced, and therefore, the production method of the present disclosure may increase the poly-4-hydroxybutyrate productivity. In addition, the present disclosure may increase the poly-4-hydroxybutyrate productivity by activating the reductive TCA cycle using oxaloacetate; by a high-productivity fermentation process; and/or by recycling carbon dioxide generated from fermentation through the reductive TCA cycle, by introducing a gene in the producing poly-4-hydroxybutyrate. In addition, productivity of 1,4-butanediol may be further increased according to the increased poly-4-hydroxybutyrate productivity.

The method of producing 1 ,4-butanediol of the present disclosure may further include any one or more cycles selected from the group consisting of the TCA cycle, the reductive TCA cycle, and the glyoxylate cycle. The cycle may lead to conversion to succinyl-CoA.

In one embodiment, the method of producing 1,4-butanediol of the present disclosure may include the TCA cycle. At this time, one molecule of glucose may be converted to pyruvate through the glycolysis cycle, which may be converted to succinyl-CoA through the TCA cycle.

The TCA cycle may include any one or more selected from the group consisting of (a1) converting pyruvate to acetyl-CoA; (b1) converting acetyl-CoA and oxaloacetate to citrate; (c1) converting citrate to isocitrate; (d1) converting isocitrate to α-ketoglutarate; (e1) converting α-ketoglutarate to succinyl-CoA; and (f1) converting pyruvate to oxaloacetate.

(d1) may be converting isocitrate to α-ketoglutarate and carbon dioxide, and (e1) may be converting α-ketoglutarate to succinyl-CoA and carbon dioxide.

In one embodiment, (a1) to (f1) may use any one or more selected from the group consisting of any one or more polypeptides selected from the group consisting of pyruvate dehydrogenase, citrate synthase, aconitase, isocitrate dehydrogenase, α-ketoglutarate dehydrogenase, and pyruvate carboxylase; a microorganism including the polypeptide, a polynucleotide encoding the polypeptide, a vector including the polynucleotide, or a combination thereof; and a culture thereof, respectively, but are not limited thereto.

The production method may further include (g1) converting phosphoenolpyruvate to oxaloacetate, but is not limited thereto.

In one embodiment, (g1) may use any one or more selected from the group consisting of a phosphoenolpyruvate carboxylase polypeptide; a microorganism including the polypeptide, a polynucleotide encoding the polypeptide, a vector including the polynucleotide, or a combination thereof; and a culture thereof, but is not limited thereto.

In one embodiment, in the production method of the present disclosure, transcription of phosphoenolpyruvate carboxylase gene (ppc) is not inhibited under limiting conditions of one or more selected from the group consisting of nitrogen, sulfur, phosphorus, and magnesium, but is not limited thereto.

In one embodiment, the production method of the present disclosure may include limiting one or more selected from the group consisting of nitrogen, phosphorus, sulfur, and magnesium, but is not limited thereto.

In one embodiment, even though the production method of the present disclosure includes limiting one or more selected from the group consisting of nitrogen, phosphorus, sulfur, and magnesium, the method may not reduce the production of poly-4-hydroxybutyrate and/or 1,4-butanediol, as compared to a method without the limiting step, but is not limited thereto.

In one embodiment, the transcription may not be inhibited by a promoter, but is not limited thereto. The promoter may be a polynucleotide having promoter activity, which is represented by SEQ ID NO: 45, and a target gene of the nucleotide sequence having the promoter activity, which is represented by SEQ ID NO: 45, may be a polynucleotide encoding the phosphoenolpyruvate carboxylase. Under nitrogen-limiting conditions, when a promoter, whereby ppc transcription is not inhibited, is used, the rTCA cycle may be enhanced and the production of poly-4-hydroxybutyrate and/or 1,4-butanediol may be increased. Under nitrogen-limiting conditions, when a promoter, whereby the phosphoenolpyruvate carboxylase gene (ppc) transcription is not inhibited, while having activity equivalent to or higher than that of the wild-type ppc promoter, it may be effective for the production of poly-4-hydroxybutyrate and/or 1,4-butanediol.

In one embodiment, in the production method, the (g1) step may be enhanced, but is not limited thereto.

In one embodiment, the method of producing 1,4-butanediol of the present disclosure may include the reductive TCA cycle. Through the reductive TCA cycle, oxaloacetate does not undergo the decarboxylation process included in the oxidative TCA cycle, and thus oxaloacetate may be converted to succinyl-CoA through malate, fumarate, and succinate without generating additional carbon dioxide.

The reductive TCA cycle may include any one or more selected from the group consisting of (a2) converting oxaloacetate to malate; (b2) converting malate to fumarate; (c2) converting fumarate to succinate; and (d2) converting succinate to succinyl-CoA, but is not limited thereto.

The production method of the present disclosure may further include (e2) converting phosphoenolpyruvate to oxaloacetate before the reductive TCA cycle. (e2) may be the same as (g1).

In one embodiment, in the production method of the present disclosure, the reductive TCA cycle may be enhanced, and the enhancement of the reductive TCA cycle may include enhancement of (e2) the converting phosphoenolpyruvate to oxaloacetate, but is not limited thereto.

In one embodiment, the reductive TCA cycle may be enhanced by any one or more selected from the group consisting of the following (I) to (XII), but is not limited thereto:
(I) weakening of pyruvate kinase; (II) enhancement of phosphoenolpyruvate carboxylase (PEP carboxylase); (III) enhancement of carbonic anhydrase; (IV) regulation of citrate synthase; (V) enhancement of pyruvate carboxylase; (VI) weakening of **NAD⁺**-dependent malate dehydrogenase; (VII) weakening of **NADP⁺**-dependent malate dehydrogenase; (VIII) weakening of phosphogluconate dehydratase; (IX) weakening of 2-keto-4-hydroxyglutarate:2-keto-3-deoxygluconate 6-phosphate aldolase (KHG/KDPG aldolase); (X) weakening of aspartate aminotransferase; (XI) weakening of glucose-specific PTS enzyme IIBC component; and (XII) enhancement of bicarbonate transporter.

In one embodiment, the converting phosphoenolpyruvate to oxaloacetate may be enhanced by weakening of pyruvate kinase and enhancement of carbonic anhydrase; weakening of pyruvate kinase and enhancement of phosphoenolpyruvate carboxylase; regulation of citrate synthase and enhancement of phosphoenolpyruvate carboxylase; regulation of citrate synthase and enhancement of carbonic anhydrase; and weakening of pyruvate kinase, enhancement of phosphoenolpyruvate carboxylase, and enhancement of pyruvate carboxylase, optionally, may be enhanced by any one or more selected from the group consisting of enhancement of carbonic anhydrase, weakening of **NAD⁺**-dependent malate dehydrogenase, weakening of **NADP⁺**-dependent malate dehydrogenase, weakening of phosphogluconate dehydratase, weakening of KHG/KDPG aldolase, weakening of aspartate aminotransferase, weakening of glucose-specific PTS enzyme IIBC component; and/or enhancement of bicarbonate transporter, but is not limited thereto.

In one embodiment, the reductive TCA cycle may include (II) enhancement of phosphoenolpyruvate carboxylase; (VI) weakening of **NAD⁺**-dependent malate dehydrogenase; (VII) weakening of **NADP⁺**-dependent malate dehydrogenase; and/or (X) weakening of aspartate aminotransferase.

In one embodiment, in the reductive TCA cycle, (II) phosphoenolpyruvate carboxylase may be enhanced.

In one embodiment, in the reductive TCA cycle, (VI) **NAD⁺**-dependent malate dehydrogenase and (VII) **NADP⁺**-dependent malate dehydrogenase may be weakened.

In one embodiment, in the reductive TCA cycle, (X) aspartate aminotransferase may be weakened.

In one embodiment, the production yield of poly-4-hydroxybutyrate and/or 1,4-butanediol may be increased by recycling carbon dioxide generated in fermentation through the rTCA cycle of the present disclosure.

In one embodiment, the method of producing 1,4-butanediol of the present disclosure may include a glyoxylate cycle.

The glyoxylate cycle may further include any one or more selected from the group consisting of (a3) converting isocitrate to glyoxylate and succinate; (b3) converting glyoxylate and acetyl-CoA to malate and CoA; (c3) converting citrate to isocitrate; (d3) converting pyruvate to oxaloacetate; (e3) converting phosphoenolpyruvate to oxaloacetate; (f3) converting oxaloacetate and acetyl-CoA to citrate; (g3) converting malate to fumarate; (h3) converting fumarate to succinate; and (i3) converting succinate to succinyl-CoA, but is not limited thereto. (f3) may be the same as (b1), (g3) may be the same as (b2), (h3) may be the same as (c2), and (i3) may be the same as (d2).

In one embodiment, the glyoxylate cycle may be by any one or more selected from the group consisting of (i) enhancement of citrate synthase; (ii) weakening of isocitrate dehydrogenase; (iii) enhancement of isocitrate lyase; (iv) enhancement of isocitrate dehydrogenase kinase/phosphatase; (v) enhancement of malate synthase G; and (vi) enhancement of malate synthase A, but is not limited thereto.

In one embodiment, in the method including the glyoxylate cycle of the present disclosure, (j3) converting α-ketoglutarate to succinyl-CoA and/or (k3) converting oxaloacetate to malate may be further weakened, but is not limited thereto.

Both succinate and malate which are products of the glyoxylate cycle may be converted to succinyl-CoA using the reductive TCA cycle.

As used herein, the "succinate semialdehyde dehydrogenase" is an enzyme capable of catalyzing conversion of succinyl-CoA (SuCoA) to succinate semialdehyde (SSA). The succinate semialdehyde dehydrogenase may be used interchangeably with SucD.

In one embodiment, the SucD protein of the present disclosure may be derived from *Clostridium kluyveri,* and any one is included in SucD as long as it has a sequence or activity equivalent thereto. In one embodiment, the SucD protein of the present disclosure may include, have, or consist of SEQ ID NO: 1 or an amino acid sequence having 80% or more homology or identity thereto, or may essentially consist of the amino acid sequence.

In the present disclosure, the succinate semialdehyde dehydrogenase gene may be used interchangeably with *sucD,* a polynucleotide encoding the succinate semialdehyde dehydrogenase, *etc.* The *sucD* gene may include, for example, a base sequence of SEQ ID NO: 2, and may consist of a base sequence having 80% or more homology or identity thereto, but is not limited thereto.

In the present disclosure, the "4-hydroxybutyric acid dehydrogenase" is an enzyme capable of catalyzing conversion of succinate semialdehyde (SSA) to 4-hydroxybutyrate (4HB). The 4-hydroxybutyric acid dehydrogenase may be used interchangeably with succinate semialdehyde reductase and 4HbD.

In one embodiment, the 4HbD protein of the present disclosure may be derived from *Arabidopsis thaliana,* and any one is included in 4HbD as long as it has a sequence or activity equivalent thereto. In one embodiment, the 4HbD protein of the present disclosure may include, have, or consist of SEQ ID NO: 3 or an amino acid sequence having 80% or more homology or identity thereto, or may essentially consist of the amino acid sequence.

In the present disclosure, the 4-hydroxybutyric acid dehydrogenase gene may be used interchangeably with *4hbD,* a polynucleotide encoding the 4-hydroxybutyric acid dehydrogenase, *etc.* The *4hbD* gene may include, for example, a base sequence of SEQ ID NO: 4, and may consist of a base sequence having 80% or more homology or identity thereto, but is not limited thereto.

In the present disclosure, the "4-hydroxybutyryl-CoA transferase" is an enzyme capable of catalyzing conversion of 4-hydroxybutyrate (4HB) to 4-hydroxybutyryl CoA (4HBCoA). The 4-hydroxybutyryl-CoA transferase may be used interchangeably with OrfZ.

In one embodiment, the OrfZ protein of the present disclosure may be derived from *Clostridium kluyveri,* and any one is included in OrfZ as long as it has a sequence or activity equivalent thereto. In one embodiment, the OrfZ protein of the present disclosure may include, have, or consist of SEQ ID NO: 5 or an amino acid sequence having 80% or more homology or identity thereto, or may essentially consist of the amino acid sequence.

In the present disclosure, the 4-hydroxybutyryl-CoA transferase gene may be used interchangeably with *orfZ,* a polynucleotide encoding the 4-hydroxybutyryl-CoA transferase, *etc.* The *orfZ* gene may include, for example, a base sequence of SEQ ID NO: 6, and may consist of a base sequence having 80% or more homology or identity thereto, but is not limited thereto.

In one embodiment, the amino acid and gene sequences of the SucD, 4HbD, and/or OrfZ may be obtained from US 9084467 B2, but are not limited thereto.

In the present disclosure, the "poly(3-hydroxyalkanoate) polymerase" is an enzyme capable of catalyzing polymerization of two or more of 4-hydroxybutyryl CoA (4HBCoA) to poly-4-hydroxybutyrate (P4HB). The poly(3-hydroxyalkanoate) polymerase may be used interchangeably with PhaC.

In one embodiment, the PhaC protein of the present disclosure may be derived from various microorganisms, specifically, *Pseudomonas putida* or *Ralstonia eutropha,* and may be a fusion protein thereof, and any one is included in PhaC as long as it has a sequence or activity equivalent thereto.

In one embodiment, the PhaC protein of the present disclosure may include, have, or consist of SEQ ID NO: 7 or an amino acid sequence having 80% or more homology or identity thereto, or may essentially consist of the amino acid sequence.

In one embodiment, the amino acid sequence and gene sequence of the PhaC may be obtained from WO 2014058655 A1, but are not limited thereto.

In the present disclosure, the poly(3-hydroxyalkanoate) polymerase gene may be used interchangeably with *phaC,* a polynucleotide encoding the poly(3-hydroxyalkanoate) polymerase, *etc.* The *phaC* gene may include, for example, a base sequence of SEQ ID NO: 8, and may consist of a base sequence having 80% or more homology or identity thereto, but is not limited thereto.

In the present disclosure, the pyruvate dehydrogenase means an enzyme capable of catalyzing conversion of pyruvate to acetyl-CoA; the citrate synthase means an enzyme capable of catalyzing production of citrate by condensation of oxaloacetate and acetyl-CoA; the aconitase means an enzyme capable of catalyzing conversion of citrate to isocitrate; the isocitrate dehydrogenase means an enzyme capable of catalyzing conversion of isocitrate to α-ketoglutarate; the α-ketoglutarate dehydrogenase means an enzyme capable of catalyzing conversion of α-ketoglutarate to succinyl-CoA; the succinyl-CoA synthetase means an enzyme capable of catalyzing conversion of succinyl-CoA to succinate; and the pyruvate carboxylase means an enzyme capable of catalyzing conversion of pyruvate to oxaloacetate.

The pyruvate dehydrogenase to pyruvate carboxylase may be enzymes included in the TCA cycle, and may be endogenous to the microorganisms or production methods, or may be enhanced, as compared to wild-type enzymes.

In the present disclosure, the "pyruvate kinase" is an enzyme capable of catalyzing conversion of phosphoenolpyruvate (PEP) to pyruvate. The pyruvate kinase may be used interchangeably with Pyk.

In one embodiment, the Pyk protein of the present disclosure may be endogenous or may be derived from the genus *Escherichia, Escherichia coli,* and any one is included in Pyk as long as it has a sequence or activity equivalent thereto.

In one embodiment, the Pyk protein of the present disclosure may be weakened, and any one is included in Pyk as long as it has a sequence or activity equivalent thereto. In one embodiment, the Pyk protein of the present disclosure may include, have, or consist of SEQ ID NO: 9, SEQ ID NO: 11, or an amino acid sequence having 80% or more homology or identity thereto, or may essentially consist of the amino acid sequence.

In the present disclosure, the pyruvate kinase gene may be used interchangeably with *pykA, pykF,* a polynucleotide encoding the pyruvate kinase, *etc.* The *pykA, pykF* gene may include, for example, a base sequence of SEQ ID NO: 10 or SEQ ID NO: 12, and may consist of a base sequence having 80% or more homology or identity thereto, but is not limited thereto.

In the present disclosure, the "phosphoenolpyruvate carboxylase (PEP carboxylase)" is an enzyme capable of catalyzing conversion of phosphoenolpyruvate to oxaloacetate. The phosphoenolpyruvate carboxylase may be used interchangeably with PPC.

In one embodiment, the PPC protein of the present disclosure may be endogenous or may be derived from the genus *Escherichia, Escherichia coli,* and any one is included in PPC as long as it has a sequence or activity equivalent thereto.

In one embodiment, the PPC protein of the present disclosure may be enhanced, and any one is included in PPC as long as it has a sequence or activity equivalent thereto. In one embodiment, the PPC protein of the present disclosure may include, have, or consist of SEQ ID NO: 13 or an amino acid sequence having 80% or more homology or identity thereto, or may essentially consist of the amino acid sequence.

In the present disclosure, the phosphoenolpyruvate carboxylase gene may be used interchangeably with *ppc,* a polynucleotide encoding the phosphoenolpyruvate carboxylase, *etc.* The *ppc* gene may include, for example, a base sequence of SEQ ID NO: 14, and may consist of a base sequence having 80% or more homology or identity thereto, but is not limited thereto.

In one embodiment, the microorganism of the present disclosure may have the poly-4-hydroxybutyrate production ability, particularly, the enhanced production ability thereof under limiting conditions of any one or more nutrients selected from the group consisting of nitrogen, sulfur, phosphorus, and magnesium, but is not limited thereto.

In one embodiment, the microorganism of the present disclosure may further include a promoter, whereby transcription of phosphoenolpyruvate carboxylase gene (ppc) is not inhibited under nitrogen, sulfur, phosphorous, and/or magnesium-limiting conditions. The promoter may be a polynucleotide having promoter activity, which is represented by SEQ ID NO: 45, and a target gene of the nucleotide sequence having the promoter activity, which is represented by SEQ ID NO: 45, may be a polynucleotide encoding the phosphoenolpyruvate carboxylase. Under nitrogen-limiting conditions, when a promoter, whereby ppc transcription is not inhibited, is used, the production of poly-4-hydroxybutyrate and/or 1,4-butanediol may be increased. The ppc expression of *E.coli* is inhibited under nitrogen-limiting conditions. In contrast, when a promoter, whereby the phosphoenolpyruvate carboxylase gene (ppc) transcription is not inhibited, while having activity equivalent to or higher than that of the wild-type ppc promoter, it may be effective for the production of poly-4-hydroxybutyrate and/or 1,4-butanediol.

In the present disclosure, the "carbonic anhydrase" is an enzyme capable of catalyzing conversion of hydrogen carbonate to carbon dioxide and water. The carbonic anhydrase may be an enzyme that plays an auxiliary role of phosphoenolpyruvate carboxylase. Bicarbonate (HCO³⁻) is required for smooth performance of PPC. The carbonic anhydrase may be an enzyme capable of producing HCO³⁻ from carbon dioxide. The carbonic anhydrase may be used interchangeably with EcaA.

In one embodiment, the EcaA protein of the present disclosure may be derived from *Nostoc* sp., and any one is included in EcaA as long as it has a sequence or activity equivalent thereto.

In one embodiment, the EcaA protein of the present disclosure may be enhanced, and any one is included in EcaA as long as it has a sequence or activity equivalent thereto. In one embodiment, the EcaA protein of the present disclosure may include, have, or consist of SEQ ID NO: 15 or an amino acid sequence having 80% or more homology or identity thereto, or may essentially consist of the amino acid sequence.

In the present disclosure, the carbonic anhydrase gene may be used interchangeably with ecaA and a polynucleotide encoding the carbonic anhydrase, *etc.* The ecaA gene may include, for example, a base sequence of SEQ ID NO: 16, and may consist of a base sequence having 80% or more homology or identity thereto, but is not limited thereto.

In the present disclosure, the "citrate synthase" may be an enzyme capable of catalyzing production of citrate by condensation of oxaloacetate and acetyl-CoA. The citrate synthase may be used interchangeably with GltA.

In one embodiment, the GltA protein of the present disclosure may be endogenous or may be derived from the genus *Escherichia, Escherichia coli,* and any one is included in GltA as long as it has a sequence or activity equivalent thereto.

In one embodiment, the GltA protein of the present disclosure may be regulated, and any one is included in GltA as long as it has a sequence or activity equivalent thereto. In one embodiment, the GltA protein of the present disclosure may include, have, or consist of SEQ ID NO: 17 or an amino acid sequence having 80% or more homology or identity thereto, or may essentially consist of the amino acid sequence.

In the present disclosure, the citrate synthase gene may be used interchangeably with *gltA* and a polynucleotide encoding the citrate synthase, *etc.* The *gltA* gene may include, for example, a base sequence of SEQ ID NO: 18, and may consist of a base sequence having 80% or more homology or identity thereto, but is not limited thereto.

In the present disclosure, the "pyruvate carboxylase" may be an enzyme capable of catalyzing conversion of pyruvate and carbon dioxide to oxaloacetate. The pyruvate carboxylase may be used interchangeably with Pyc.

In one embodiment, the Pyc protein of the present disclosure may be derived from *Rhizobium etli,* and any one is included in Pyc as long as it has a sequence or activity equivalent thereto.

In one embodiment, the Pyc protein of the present disclosure may be enhanced and may be exogenous, and any one is included in Pyc as long as it has a sequence or activity equivalent thereto. In one embodiment, the Pyc protein of the present disclosure may include, have, or consist of SEQ ID NO: 19 or an amino acid sequence having 80% or more homology or identity thereto, or may essentially consist of the amino acid sequence.

In the present disclosure, the pyruvate carboxylase gene may be used interchangeably with *pyc* and a polynucleotide encoding the pyruvate carboxylase, *etc.* The *pyc* gene may include, for example, a base sequence of SEQ ID NO: 20, and may consist of a base sequence having 80% or more homology or identity thereto, but is not limited thereto.

In the present disclosure, the "**NAD⁺**-dependent malate dehydrogenase" may be an enzyme capable of catalyzing conversion of malate to pyruvate. The **NAD⁺**-dependent malate dehydrogenase may be used interchangeably with MaeA.

In one embodiment, the MaeA protein of the present disclosure may be endogenous or may be derived from the genus *Escherichia, Escherichia coli,* and any one is included in MaeA as long as it has a sequence or activity equivalent thereto.

In one embodiment, the MaeA protein of the present disclosure may be weakened, and any one is included in MaeA as long as it has a sequence or activity equivalent thereto. In one embodiment, the MaeA protein of the present disclosure may include, have, or consist of SEQ ID NO: 21 or an amino acid sequence having 80% or more homology or identity thereto, or may essentially consist of the amino acid sequence.

In the present disclosure, the **NAD⁺**-dependent malate dehydrogenase gene may be used interchangeably with *maeA* and a polynucleotide encoding the **NAD⁺**-dependent malate dehydrogenase, *etc.* The *maeA* gene may include, for example, a base sequence of SEQ ID NO: 22, and may consist of a base sequence having 80% or more homology or identity thereto, but is not limited thereto.

In the present disclosure, the "**NADP⁺**-dependent malate dehydrogenase" may be an enzyme capable of catalyzing conversion of malate to pyruvate. The **NADP⁺-**dependent malate dehydrogenase may be used interchangeably with MaeB.

In one embodiment, the MaeB protein of the present disclosure may be endogenous or may be derived from the genus *Escherichia, Escherichia coli,* and any one is included in MaeB as long as it has a sequence or activity equivalent thereto.

In one embodiment, the MaeB protein of the present disclosure may be weakened, and any one is included in MaeB as long as it has a sequence or activity equivalent thereto. In one embodiment, the MaeB protein of the present disclosure may include, have, or consist of SEQ ID NO: 23 or an amino acid sequence having 80% or more homology or identity thereto, or may essentially consist of the amino acid sequence.

In the present disclosure, the **NADP⁺**-dependent malate dehydrogenase gene may be used interchangeably with *mae8* and a polynucleotide encoding the **NADP⁺**-dependent malate dehydrogenase, *etc.* The *mae8* gene may include, for example, a base sequence of SEQ ID NO: 24, and may consist of a base sequence having 80% or more homology or identity thereto, but is not limited thereto.

In the present disclosure, the "phosphogluconate dehydratase" may be an enzyme capable of catalyzing conversion of 6-phospho-D-gluconate to 2-dehydro-3-deoxy-6-phospho-D-gluconate. The phosphogluconate dehydratase may be used interchangeably with EDD.

In one embodiment, the EDD protein of the present disclosure may be endogenous or may be derived from the genus *Escherichia, Escherichia coli,* and any one is included in EDD as long as it has a sequence or activity equivalent thereto.

In one embodiment, the EDD protein of the present disclosure may be weakened, and any one is included in EDD as long as it has a sequence or activity equivalent thereto. In one embodiment, the EDD protein of the present disclosure may include, have, or consist of SEQ ID NO: 25 or an amino acid sequence having 80% or more homology or identity thereto, or may essentially consist of the amino acid sequence.

In the present disclosure, the phosphogluconate dehydratase gene may be used interchangeably with edd and a polynucleotide encoding the phosphogluconate dehydratase, *etc.* The edd gene may include, for example, a base sequence of SEQ ID NO: 26, and may consist of a base sequence having 80% or more homology or identity thereto, but is not limited thereto.

In the present disclosure, the "2-keto-4-hydroxyglutarate:2-keto-3-deoxygluconate 6-phosphate aldolase (KHG/KDPG aldolase)" is an enzyme capable of catalyzing conversion of 4-hydroxy-2-oxoglutarate to glyceraldehyde-3-phosphate and pyruvate. The phosphogluconate dehydratase may be used interchangeably with KHG/KDPG aldolase or Eda.

In one embodiment, the Eda protein of the present disclosure may be endogenous or may be derived from the genus *Escherichia, Escherichia coli,* and any one is included in Eda as long as it has a sequence or activity equivalent thereto.

In one embodiment, the Eda protein of the present disclosure may be weakened, and any one is included in Eda as long as it has a sequence or activity equivalent thereto. In one embodiment, the Eda protein of the present disclosure may include, have, or consist of SEQ ID NO: 27 or an amino acid sequence having 80% or more homology or identity thereto, or may essentially consist of the amino acid sequence.

In the present disclosure, the KHG/KDPG aldolase gene may be used interchangeably with eda and a polynucleotide encoding the KHG/KDPG aldolase, *etc.* The eda gene may include, for example, a base sequence of SEQ ID NO: 28, and may consist of a base sequence having 80% or more homology or identity thereto, but is not limited thereto.

In the present disclosure, the "aspartate aminotransferase" is an enzyme capable of catalyzing conversion of 2-oxoglutarate and aspartate to glutamate and oxaloacetate. The aspartate aminotransferase may be used interchangeably with AspC.

In one embodiment, the AspC protein of the present disclosure may be endogenous or may be derived from the genus *Escherichia, Escherichia coli,* and any one is included in AspC as long as it has a sequence or activity equivalent thereto.

In one embodiment, the AspC protein of the present disclosure may be weakened, and any one is included in AspC as long as it has a sequence or activity equivalent thereto. In one embodiment, the AspC protein of the present disclosure may include, have, or consist of SEQ ID NO: 29 or an amino acid sequence having 80% or more homology or identity thereto, or may essentially consist of the amino acid sequence.

In the present disclosure, the aspartate aminotransferase gene may be used interchangeably with *aspC* and a polynucleotide encoding the aspartate aminotransferase, *etc.* The *aspC* gene may include, for example, a base sequence of SEQ ID NO: 30, and may consist of a base sequence having 80% or more homology or identity thereto, but is not limited thereto.

In the present disclosure, the "glucose-specific PTS enzyme IIBC component (glucose-specific phosphoenolpyruvate-dependent sugar phosphotransferase system (PTS) enzyme IIBC component)" is a part of PTS which is an enzyme involved in glucose transport. In one embodiment, the glucose-specific PTS enzyme IIBC component may enhance the reductive TCA cycle by increasing a phosphoenolpyruvate pool. The glucose-specific PTS enzyme IIBC component may be used interchangeably with IIBC.

In one embodiment, the IIBC protein of the present disclosure may be endogenous or may be derived from the genus *Escherichia, Escherichia coli,* and any one is included in IIBC as long as it has a sequence or activity equivalent thereto.

In one embodiment, the IIBC protein of the present disclosure may be weakened, and any one is included in IIBC as long as it has a sequence or activity equivalent thereto. In one embodiment, the IIBC protein of the present disclosure may include, have, or consist of SEQ ID NO: 31 or an amino acid sequence having 80% or more homology or identity thereto, or may essentially consist of the amino acid sequence.

In the present disclosure, the glucose-specific PTS enzyme IIBC component gene may be used interchangeably with *ptsG* and a polynucleotide encoding the glucose-specific PTS enzyme IIBC component, *etc.* The *ptsG* gene may include, for example, a base sequence of SEQ ID NO: 32, and may consist of a base sequence having 80% or more homology or identity thereto, but is not limited thereto.

In the present disclosure, the "bicarbonate transporter" is a transporter transporting bicarbonate, and may increase intracellular uptake of carbon dioxide. The bicarbonate transporter may be used interchangeably with SbtA.

In one embodiment, the SbtA protein of the present disclosure may be derived from *Synechocystis* sp., and any one is included in SbtA as long as it has a sequence or activity equivalent thereto.

In one embodiment, the SbtA protein of the present disclosure may be enhanced, may be exogenous, and may be derived from *Cyanobacteria,* and any one is included in SbtA as long as it has a sequence or activity equivalent thereto. In one embodiment, the SbtA protein of the present disclosure may include, have, or consist of SEQ ID NO: 33 or an amino acid sequence having 80% or more homology or identity thereto, or may essentially consist of the amino acid sequence.

In the present disclosure, the SbtA gene may be used interchangeably with *sbtA* and a polynucleotide encoding the bicarbonate transporter, *etc.* The *sbtA* gene may include, for example, a base sequence of SEQ ID NO: 34, and may consist of a base sequence having 80% or more homology or identity thereto, but is not limited thereto.

In the present disclosure, "isocitrate dehydrogenase" is an enzyme capable of catalyzing conversion of isocitrate to 2-oxoglutarate. The isocitrate dehydrogenase may be used interchangeably with Icd.

In one embodiment, the Icd protein of the present disclosure may be endogenous or may be derived from the genus *Escherichia, Escherichia coli,* and any one is included in Icd as long as it has a sequence or activity equivalent thereto.

In one embodiment, the Icd protein of the present disclosure may be weakened, and any one is included in Icd as long as it has a sequence or activity equivalent thereto. In one embodiment, the Icd protein of the present disclosure may include, have, or consist of SEQ ID NO: 35 or an amino acid sequence having 80% or more homology or identity thereto, or may essentially consist of the amino acid sequence.

In the present disclosure, the Icd gene may be used interchangeably with *icd* and a polynucleotide encoding the isocitrate dehydrogenase, *etc.* The *icd* gene may include, for example, a base sequence of SEQ ID NO: 36, and may consist of a base sequence having 80% or more homology or identity thereto, but is not limited thereto.

In the present disclosure, "isocitrate lyase" is an enzyme capable of catalyzing conversion of isocitrate to glyoxylate and succinate. The isocitrate lyase may be used interchangeably with AceA.

In one embodiment, the AceA protein of the present disclosure may be endogenous or may be derived from the genus *Escherichia, Escherichia coli,* and any one is included in AceA as long as it has a sequence or activity equivalent thereto.

In one embodiment, the AceA protein of the present disclosure may be enhanced, and any one is included in AceA as long as it has a sequence or activity equivalent thereto. In one embodiment, the AceA protein of the present disclosure may include, have, or consist of SEQ ID NO: 37 or an amino acid sequence having 80% or more homology or identity thereto, or may essentially consist of the amino acid sequence.

In the present disclosure, the AceA gene may be used interchangeably with aceA and a polynucleotide encoding the isocitrate lyase, *etc.* The *aceA* gene may include, for example, a base sequence of SEQ ID NO: 38, and may consist of a base sequence having 80% or more homology or identity thereto, but is not limited thereto.

In the present disclosure, "isocitrate dehydrogenase kinase/phosphatase" is an enzyme capable of catalyzing phosphorylation or dephosphorylation of isocitrate dehydrogenase. In one embodiment, the isocitrate dehydrogenase kinase/phosphatase may weaken Icd. The isocitrate dehydrogenase kinase/phosphatase may be used interchangeably with AceK.

In one embodiment, the AceK protein of the present disclosure may be endogenous or may be derived from the genus *Escherichia, Escherichia coli,* and any one is included in AceK as long as it has a sequence or activity equivalent thereto.

In one embodiment, the AceK protein of the present disclosure may be enhanced, and any one is included in AceK as long as it has a sequence or activity equivalent thereto. In one embodiment, the AceK protein of the present disclosure may include, have, or consist of SEQ ID NO: 39 or an amino acid sequence having 80% or more homology or identity thereto, or may essentially consist of the amino acid sequence.

In the present disclosure, the AceK gene may be used interchangeably with *aceK* and a polynucleotide encoding the isocitrate dehydrogenase kinase/phosphatase, *etc.* The *aceK* gene may include, for example, a base sequence of SEQ ID NO: 40, and may consist of a base sequence having 80% or more homology or identity thereto, but is not limited thereto.

In the present disclosure, the "malate synthase G" is an enzyme capable of catalyzing conversion of glyoxylate and acetyl-CoA to malate. The malate synthase G may be used interchangeably with GlcB.

In one embodiment, the GlcB protein of the present disclosure may be endogenous or may be derived from the genus *Escherichia, Escherichia coli,* and any one is included in GlcB as long as it has a sequence or activity equivalent thereto.

In one embodiment, the GlcB protein of the present disclosure may be enhanced, and any one is included in GlcB as long as it has a sequence or activity equivalent thereto. In one embodiment, the GlcB protein of the present disclosure may include, have, or consist of SEQ ID NO: 41 or an amino acid sequence having 80% or more homology or identity thereto, or may essentially consist of the amino acid sequence.

In the present disclosure, the GlcB gene may be used interchangeably with *glcB* and a polynucleotide encoding the malate synthase G, *etc.* The *glcB* gene may include, for example, a base sequence of SEQ ID NO: 42, and may consist of a base sequence having 80% or more homology or identity thereto, but is not limited thereto.

In the present disclosure, the "malate synthase A" is an enzyme capable of catalyzing conversion of glyoxylate and acetyl-CoA to malate. The malate synthase A may be used interchangeably with AceB.

In one embodiment, the AceB protein of the present disclosure may be endogenous or may be derived from the genus *Escherichia, Escherichia coli,* and any one is included in AceB as long as it has a sequence or activity equivalent thereto.

In one embodiment, the AceB protein of the present disclosure may be enhanced, and any one is included in AceB as long as it has a sequence or activity equivalent thereto. In one embodiment, the AceB protein of the present disclosure may include, have, or consist of SEQ ID NO: 43 or an amino acid sequence having 80% or more homology or identity thereto, or may essentially consist of the amino acid sequence.

In the present disclosure, the AceB gene may be used interchangeably with *aceB* and a polynucleotide encoding the malate synthase A, *etc*. The *aceB* gene may include, for example, a base sequence of SEQ ID NO: 44, and may consist of a base sequence having 80% or more homology or identity thereto, but is not limited thereto.

Amino acid sequences of the above enzymes (e.g., SucD, *etc.)* may be obtained from various databases, such as GenBank of NCBI, *etc.,* which is a known database, but are not limited thereto.

In the present disclosure, although expressed as a "polypeptide or protein including an amino acid sequence described by a specific sequence number", a "polypeptide or protein consisting of an amino acid sequence described by a specific sequence number", or a "polypeptide or protein having an amino acid sequence described by a specific sequence number", it is obvious that any protein including deletion, modification, substitution, conservative substitution, or addition in part of the sequence may be used in the present disclosure as long as the protein has activity identical or equivalent to that of the polypeptide consisting of the amino acid sequence of the corresponding sequence number, for example, those having addition of a sequence that do not alter the function of the protein, at the N-terminus, inside, and/or C-terminus of the amino acid sequence, or a naturally occurring mutation, a silent mutation, or a conservative substitution.

Specifically, the protein of the present disclosure may include an amino acid sequence of a specific sequence number, or an amino acid sequence having at least 80%, 85%, 86%, 87%, 88%, 89%, 90%, 91%, 92%, 93%, 94%, 95%, 96%, 97%, 98% or 99% homology or identity to the amino acid sequence of the specific sequence number. Further, it is apparent that any protein having an amino acid sequence with deletion, modification, substitution, or addition in part of the sequence may also belong to the scope of the present disclosure, as long as the amino acid sequence has the homology or identity and exhibits efficacy corresponding to the above protein.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, conservative substitution may hardly affect or not affect the activity of the polypeptides.

As used herein, the term "homology" or "identity" means the degree of identity or similarity between two given amino acid sequences or base sequences and may be expressed as a percentage. The terms "homology" and "identity" may often be used interchangeably.

The sequence homology or identity of a conserved polynucleotide or polypeptide is determined by standard alignment algorithms, and the default gap penalty established by the used program may be used together. Substantially, homologous or identical sequences are generally capable of being hybridized with the entirety or at least 50%, 60%, 70%, 80%, or 90% of the entire length under moderately or highly stringent conditions. It is apparent that hybridization also includes hybridization of a polynucleotide with a polynucleotide including a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al. (1988) Proc. Natl. Acad. Sci. USA 85:2444. Alternatively, it may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package ((Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443, as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al. (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

Additionally, whether any two polynucleotide or polypeptide sequences have homology, similarity, or identity may be determined by comparing these sequences via Southern hybridization experiments under defined stringent conditions, and the appropriate hybridization conditions to be defined may be within the scope of the technology and may be determined by a method well known to one of ordinary skill in the art (e.g., J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York).

The gene of the present disclosure (*e.g.*, *sucD*) may be obtained from various databases, such as GenBank of NCBI, *etc.,* which is a known database, but are not limited thereto.

For example, the *sucD* gene derived from *Clostridium kluyveri* may include, have, or consist of the base sequence of SEQ ID NO: 2; the *4hbD* gene derived from *Arabidopsis thaliana* may include, have, or consist of the base sequence of SEQ ID NO: 4; the *orfZ* gene derived from *Clostridium kluyveri* may include, have, or consist of the base sequence of SEQ ID NO: 6; the poly(3-hydroxyalkanoate) polymerase fusion protein *phaC3*/*C1* gene derived from *Pseudomonas putidalRalstonla eutropha* may include, have, or consist of the base sequence of SEQ ID NO: 8; the *pykA, pykF, ppc, gltA, maeA, mae8, edd, eda, ptsG, icd,* aceA, *aceK, g*/*cB,* and *aceB* genes derived from *Escherichia coli* may include, have, or consist of the base sequences of SEQ ID NOS: 10, 12, 14, 18, 22, 24, 26, 28, 30, 32, 36, 38, 40, 42, and 44, respectively; the ecaA gene derived from *Nostoc* sp. may include, have, or consist of the base sequence of SEQ ID NO: 16; the *pyc* gene derived from derived from *Rhizobium etli* may include, have, or consist of the base sequence of SEQ ID NO: 20; and the *sbtA* gene derived from *Synechocystis* sp. may include, have, or consist of the base sequence of SEQ ID NO: 34, but are not limited thereto.

In one embodiment, the genes of the present disclosure may be codonoptimized for the microorganisms of the genus *Escherichia* or *Corynebacterium,* but are not limited thereto.

As used herein, the term "polynucleotide" is a DNA strand having a certain length or more as a polymer of nucleotides in which nucleotide monomers are connected in a long chain by covalent bonds.

In the polynucleotide or gene of the present disclosure, various modifications may be made in the coding region as long as the amino acid sequence of the polypeptide is not changed in consideration of codon degeneracy or codons preferred in organisms that are intended to express a specific polypeptide. The polynucleotide or gene may include, for example, the base sequence(s) of SEQ ID NOS: 2, 4, 6, 8, 10, 12, 14, 16, 18, 20, 22, 24, 26, 28, 30, 32, 34, 36, 38, 40, 42, and/or 44, and may consist of a base sequence having 80% or more, 90% or more, 95% or more, 96% or more, 97% or more, 98% or more, or 99% or more homology or identity thereto, but is not limited thereto.

Further, the polynucleotide or gene of the present disclosure may include a probe that may be prepared from a known gene sequence, for example, a sequence without limitation as long as it is a sequence encoding the amino acid sequence of the present discourse by hybridizing with a complementary sequence to the entirety or a part of the base sequence of the present disclosure under stringent conditions. The "stringent conditions" means conditions that enable specific hybridization between polynucleotides. These conditions are specifically described in documents (*e.g.,* J. Sambrook *et al., supra*). Examples thereof include conditions in which polynucleotides having higher homology or identity, namely, polynucleotides having 40% or more, specifically, 90% or more, more specifically, 95% or more, 96% or more, 97% or more, 98% or more, more specifically, 99% or more homology or identity are hybridized with each other while polynucleotides having lower homology or identity are not hybridized with each other, or washing conditions for common Southern hybridization, in which washing is performed once, specifically, two to three times at a salt concentration and temperature equivalent to 60°C, 1× SSC, 0.1% SDS, specifically 60°C, 0.1× SSC, 0.1% SDS, and more specifically 68°C, 0.1× SSC, 0.1% SDS.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Therefore, the polynucleotide of the present disclosure may also include substantially similar nucleic acid sequences as well as isolated nucleic acid fragments that are complementary to the entire sequence.

Specifically, a polynucleotide having homology or identity may be detected using hybridization conditions including a hybridization step at a Tₘ value of 55°C and the above-described conditions. The Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (see Sambrook *et al., supra,* 9.50-9.51, 11.7-11.8).

In the present disclosure, use of any one or more selected from the group consisting of the microorganism including the polypeptide, the polynucleotide encoding the polypeptide, or a combination thereof; and the culture thereof may be culturing of the microorganism including the polypeptide, the polynucleotide encoding the polypeptide, or a combination thereof, and/or recovering of a specific material (e.g., succinate semialdehyde, 4-hydroxybutyrate, or 4-hydroxybutyryl CoA, *etc.)* from the cultured microorganism or medium, but is not limited thereto.

In the production method of the present disclosure, the "weakening" may refer to weakening of a specific pathway or step, and weakening of an enzyme involved in the pathway or step. The weakening may be used interchangeably with the term inactivation, deficiency, down-regulation, decrease, reduce, attenuation, *etc.* The weakening of an enzyme may be decrease, deletion, or inactivation of any one or more selected from the group consisting of the enzyme polypeptide; a microorganism including the polypeptide, a polynucleotide encoding the polypeptide, a vector including the polynucleotide, or a combination thereof; and a culture thereof. Given that the production method of the present disclosure may use the microorganism, the "weakening" in the production method of the present disclosure includes "weakening of the polypeptide activity".

In the production method of the present disclosure, the "enhancing" may refer to enhancing of a specific pathway or step, and enhancing of an enzyme involved in the pathway or step. The enhancing may be used interchangeably with the term activation, up-regulation, overexpression, increase, *etc.* The enhancing of an enzyme may be increase, activation, or overexpression of any one or more selected from the group consisting of the enzyme polypeptide; a microorganism including the polypeptide, a polynucleotide encoding the polypeptide, a vector including the polynucleotide, or a combination thereof; and a culture thereof. Given that the production method of the present disclosure may use the microorganism, the "enhancing" in the production method of the present disclosure includes "enhancing of the polypeptide activity".

As used herein, the term "regulation" may refer to the "enhancing" and/or "weakening", but is not limited thereto.

In the present disclosure, (5) the degrading poly-4-hydroxybutyrate to 1,4-butanediol may employ a chemical method, and may employ pyrolysis, hydrogenation, or a combination thereof, but is not limited thereto, as long as it is able to degrade poly-4-hydroxybutyrate to 1,4-butanediol.

Another aspect of the present disclosure provides a microorganism including succinate semialdehyde dehydrogenase, 4-hydroxybutyric acid dehydrogenase, 4-hydroxybutyryl-CoA transferase, and poly(3-hydroxyalkanoate) polymerase polypeptides, polynucleotides encoding the polypeptides, a vector including the polynucleotides, or a combination thereof.

The succinate semialdehyde dehydrogenase, 4-hydroxybutyric acid dehydrogenase, 4-hydroxybutyryl-CoA transferase, and poly(3-hydroxyalkanoate) polymerase polypeptides, the polynucleotide, the vector, the following 1,4-butanediol, TCA cycle, reductive TCA cycle, pyruvate dehydrogenase, citrate synthase, aconitase, isocitrate dehydrogenase, α-ketoglutarate dehydrogenase, pyruvate carboxylase, (I) to (XII), (i) to (vi), and enzymes related thereto are as described in other aspects.

In the present disclosure, the microorganism preferentially produces cell-friendly poly-4-hydroxybutyrate, and therefore, the microorganism of the present disclosure may have the enhanced poly-4-hydroxybutyrate production ability, and accordingly, may further have the enhanced 1,4-butanediol production ability. Further, the microorganism of the present disclosure may have the enhanced poly-4-hydroxybutyrate production ability by activation of the reductive TCA cycle using oxaloacetate by introducing genes in the producing poly-4-hydroxybutyrate; by a high-productivity fermentation process; and/or by recycling of carbon dioxide generated in the fermentation through the reductive TCA cycle.

In one embodiment, the microorganism may have the enhanced activity of any one or more polypeptides selected from the group consisting of succinate semialdehyde dehydrogenase, 4-hydroxybutyric acid dehydrogenase, 4-hydroxybutyryl-CoA transferase, and poly(3-hydroxyalkanoate) polymerase.

In one embodiment, genes, each encoding the succinate semialdehyde dehydrogenase, 4-hydroxybutyric acid dehydrogenase, 4-hydroxybutyryl-CoA transferase, and/or poly(3-hydroxyalkanoate) polymerase, may be introduced from foreign sources, but are not limited thereto.

For example, the genes encoding the succinate semialdehyde dehydrogenase and/or 4-hydroxybutyryl-CoA transferase may be derived from *Clostridium kluyveri,* the gene encoding 4-hydroxybutyric acid dehydrogenase may be derived from *Arabidopsis thaliana,* but are not limited thereto.

For example, the gene encoding the poly(3-hydroxyalkanoate) polymerase may be derived from *Pseudomonas putida* or *Ralstonia eutropha,* but is not limited thereto.

In one embodiment, the microorganism may include the TCA cycle.

In one embodiment, the microorganism may include any one or more selected from the group consisting of pyruvate dehydrogenase, citrate synthase, aconitase, isocitrate dehydrogenase, α-ketoglutarate dehydrogenase, and pyruvate carboxylase, a polynucleotide encoding the same, or a combination thereof, but is not limited thereto.

In one embodiment, the microorganism of the present disclosure may include the reductive TCA cycle, which may be enhanced.

In one embodiment, the microorganism of the present disclosure may include any one or more selected from the group consisting of the following (I) to (XII), but is not limited thereto:
(I) weakening of pyruvate kinase;
(II) enhancement of phosphoenolpyruvate carboxylase;
(III) enhancement of carbonic anhydrase;
(IV) regulation of citrate synthase;
(V) enhancement of pyruvate carboxylase;
(VI) weakening of NAD⁺-dependent malate dehydrogenase;
(VII) weakening of NADP⁺-dependent malate dehydrogenase;
(VIII) weakening of phosphogluconate dehydratase;
(IX) weakening of 2-keto-4-hydroxyglutarate:2-keto-3-deoxygluconate 6-phosphate aldolase;
(X) weakening of aspartate aminotransferase;
(XI) weakening of glucose-specific PTS enzyme IIBC component; and
(XII) enhancement of bicarbonate transporter.

In one embodiment, the microorganism of the present disclosure may include
(II) enhancement of phosphoenolpyruvate carboxylase;
(VI) weakening of NAD⁺-dependent malate dehydrogenase;
(VII) weakening of NADP⁺-dependent malate dehydrogenase; and/or
(X) weakening of aspartate aminotransferase.

In one embodiment, the microorganism of the present disclosure may include (II) enhancement of phosphoenolpyruvate carboxylase.

In one embodiment, the microorganism of the present disclosure may include (VI) weakening of NAD⁺-dependent malate dehydrogenase and (VII) weakening of NADP⁺-dependent malate dehydrogenase.

In one embodiment, the microorganism of the present disclosure may include (X) weakening of aspartate aminotransferase.

In one embodiment, the gene encoding the pyruvate carboxylase may be a foreign gene, specifically, may be derived from *Rhizobium etli,* but is not limited thereto.

In one embodiment, the regulation of citrate synthase may be caused by genetic variation, but is not limited thereto.

In one embodiment, the microorganism of the present disclosure may include the glyoxylate cycle, which may be enhanced.

In one embodiment, the microorganism of the present disclosure may include any one or more selected from the group consisting of the following (i) to (vi), but is not limited thereto:
(i) enhancement of citrate synthase;
(ii) weakening of isocitrate dehydrogenase;
(iii) enhancement of isocitrate lyase;
(iv) enhancement of isocitrate dehydrogenase kinase/phosphatase;
(v) enhancement of malate synthase G; and
(vi) enhancement of malate synthase A.

The succinate semialdehyde dehydrogenase, *etc.* is as described in other aspects.

In one embodiment, the microorganism of the present disclosure may have the poly-4-hydroxybutyrate production ability even under limiting conditions of any one or more nutrients selected from the group consisting of nitrogen, sulfur, phosphorus, and magnesium, but is not limited thereto.

In one embodiment, in the microorganism of the present disclosure, transcription of the phosphoenolpyruvate carboxylase gene (ppc) may not be inhibited even under limiting conditions of any one or more nutrients selected from the group consisting of nitrogen, sulfur, phosphorus, and magnesium, but is not limited thereto.

In one embodiment, the poly-4-hydroxybutyrate production ability of the microorganism of the present disclosure may be maintained or may not be reduced even under limiting conditions of any one or more nutrients selected from the group consisting of nitrogen, sulfur, phosphorus, and magnesium, as compared to those under conditions without nutrient limitation, but is not limited thereto.

In one embodiment, the microorganism, in which the polypeptides (e.g., SucD, PhaC, OrfZ) of interest in the present disclosure are included or enhanced, may include the polypeptide of interest, a polynucleotide encoding the polypeptide, or a vector including the polynucleotide.

The vector of the present disclosure may include a DNA construct including a base sequence of a polynucleotide encoding a polypeptide of interest operably linked to a suitable expression control region (or expression control sequence) so that the polypeptide of interest may be expressed in a suitable host. The expression control region may include a promoter capable of initiating transcription, any operator sequence for regulating the transcription, a sequence encoding a suitable mRNA ribosome binding site, and a sequence regulating termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors may include natural or recombinant plasmids, cosmids, viruses, and bacteriophages. For example, pWE15, M13, MBL3, MBL4, IXII, ASHII, APII, t10, t11, Charon4A, Charon21A, or the like may be used as a phage vector or a cosmid vector. pDZ system, pBR system, pUC system, pBluescriptll system, pGEM system, pTZ system, pCL system, pET system, or the like may be used as a plasmid vector. Specifically, pDZ, pDC, pDCM2 (Korean Patent Publication No. 10-2020-0136813), pACYC177, pACYC184, pCL, pECCG117, pUC19, pBR322, pMW118, pCC1BAC, pIMR53 vector, or the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for identifying the chromosome insertion. The selection marker is for selecting the cells transformed with vectors, *i.e*., for identifying the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a host cell or a microorganism so that the polypeptide encoded by the polynucleotide may be expressed in the host cell. The transformed polynucleotide may be located by being inserted into the chromosome of the host cell or located outside the chromosome, or irrespective thereof, as long as it may be expressed in the host cell. Further, the polynucleotide includes DNA and RNA encoding a polypeptide of interest. The polynucleotide may be introduced in any form as long as it may be introduced into a host cell and then expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, as used herein, the term "operably linked" means that the polynucleotide sequence is functionally linked to a promoter sequence that initiates and mediates transcription of the polynucleotide encoding the polypeptide of interest of the present disclosure.

In one embodiment, the microorganism including the succinate semialdehyde dehydrogenase, 4-hydroxybutyric acid dehydrogenase, 4-hydroxybutyryl-CoA transferase, and poly(3-hydroxyalkanoate) polymerase polypeptides of the present disclosure, or the polynucleotides encoding the same, the vector including the polynucleotides, or a combination thereof may have the enhanced activities of succinate semialdehyde dehydrogenase, 4-hydroxybutyric acid dehydrogenase, 4-hydroxybutyryl-CoA transferase, and poly(3-hydroxyalkanoate) polymerase, as compared to microorganisms without the same, but is not limited thereto.

As used herein, the term "microorganism" or "strain" includes all wild-type microorganisms or naturally or artificially genetically modified microorganisms, and it may be a microorganism in which a specific mechanism is strengthened due to insertion of a foreign gene or an activity enhancement of an endogenous gene, *etc.*

In one embodiment, the microorganism of the present disclosure may be a microorganism of the genus *Escherichia* or the genus *Corynebacterium,* specifically, *Escherichia coli* or *Corynebacterium glutamicum,* but is not limited thereto.

The microorganism of the present disclosure may be for the production of poly-4-hydroxybutyrate, and poly-4-hydroxybutyrate produced therefrom may be used to produce 1,4-butanediol by a chemical process, and thus the microorganism of the present disclosure may be used in the production of 1,4-butanediol, but is not limited thereto.

The microorganism of the present disclosure may be a microorganism including any one or more of the polypeptide of the present disclosure to be enhanced or introduced (*e.g*., SucD, 4HbD, OrfZ, PhaC, PPC, EcaA, Glta, Pyc, AceK, and/or AceA, *etc.),* a polynucleotide encoding the polypeptide, or a vector including the polynucleotide; a microorganism modified to express the polypeptide or gene of the present disclosure to be enhanced or introduced; a microorganism expressing the polypeptide or gene of the present disclosure to be enhanced or introduced; a microorganism having the activity of the polypeptide or gene of the present disclosure; a microorganism modified such that the polypeptide *(e.g.,* Pyk, GltA, MaeA, MaeB, Edd, Eda, and/or AspC, *etc.)* of the present disclosure to be weakened is weakened; and/or a microorganism (*e.g*., recombinant strain) in which the polypeptide or gene of the present disclosure to be weakened or the activity thereof is weakened, but is not limited thereto.

The microorganism of the present disclosure may be a microorganism naturally having the ability to produce polypeptide *(e.g.,* SucD, MaeA, *etc.)* of the present disclosure, 1,4-butanediol, and/or poly-4-hydroxybutyrate; or a microorganism, in which the polypeptide, gene, polynucleotide of the present disclosure to be enhanced or introduced, or a vector including the same is introduced into a parent strain having no ability to produce the polypeptide, 1,4-butanediol, and/or poly-4-hydroxybutyrate, or the gene or polynucleotide to be weakened, or activity thereof is weakened to enhance or weaken the polypeptide, or the ability to produce 1,4-butanediol and/or poly-4-hydroxybutyrate is enhanced or provided, but is not limited thereto.

For example, the microorganism of the present disclosure may include all of microorganisms which are transformed (accordingly, enhanced, weakened, introduced, *etc.)* with the polypeptide, gene, polynucleotide of the present disclosure, or the vector including the same, thereby producing 1,4-butanediol and/or poly-4-hydroxybutyrate or having the increased productivity.

For example, the microorganism of the present disclosure may be a recombinant microorganism, in which the ability to produce 1,4-butanediol and/or poly-4-hydroxybutyrate is increased by expressing or weakening the polypeptides of the present disclosure in the natural wild-type microorganism, 1,4-butanediol and/or poly-4-hydroxybutyrate-producing microorganism. The recombinant microorganism, in which the ability to produce 1,4-butanediol and/or poly-4-hydroxybutyrate is increased, may be a microorganism in which the ability to produce 1,4-butanediol and/or poly-4-hydroxybutyrate is increased, as compared to the natural wild-type microorganism or a microorganism in which the polypeptides of the present disclosure are unmodified (*i.e*., a microorganism including the wild-type gene, a microorganism in which the genes of the present disclosure are enhanced or not introduced, or a microorganism in which the genes of the present disclosure are not weakened), but is not limited thereto.

For example, the recombinant microorganism having the increased production ability may have about 0.001% or more, or 0.01% or more increase in the ability to produce 1,4-butanediol and/or poly-4-hydroxybutyrate, as compared to the ability to produce 1,4-butanediol and/or poly-4-hydroxybutyrate of the parent strain before modification or the unmodified microorganism, but is not limited thereto, as long as the recombinant microorganism has an increased amount of + value, as compared to the production ability of the parent strain before modification or the unmodified microorganism. The term "about" refers to a range including all of ±0.5, ±0.4, ±0.3, ±0.2, ±0.1, *etc.,* and includes all of the values that are equivalent or similar to those following the term "about", but is not limited thereto.

As the term "unmodified microorganism" does not exclude microorganisms including mutations that may occur naturally in microorganisms, and may be a wild-type microorganism or a natural microorganism itself or may be a microorganism before the trait is changed by genetic variation due to natural or artificial factors. For example, the unmodified microorganism may be a microorganism, in which the polypeptides of the present disclosure are not expressed or not weakened, or before introduction thereof. The term "unmodified microorganism" may be used interchangeably with "strain before being modified", "microorganism before being modified", "unvaried strain", "unmodified strain", "unvaried microorganism", or "reference microorganism".

In the microorganism of the present disclosure, partial or entire modification of a polynucleotide may be induced by (a) homologous recombination using a vector for chromosome insertion in the microorganism or genome editing using engineered nuclease *(e.g.,* CRISPR-Cas9) and/or (b) treatment with light such as ultraviolet rays and radiation and/or chemicals, but is not limited thereto. A method of modifying a part or the entirety of the gene may include a method using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may include a dominant selection marker, but is not limited thereto.

As used herein, the term "weakening" of activity of a polypeptide is a concept including both cases where the activity is decreased, as compared to the endogenous activity, or the activity is absent. The weakening may be used interchangeably with terms such as inactivation, deficiency, down-regulation, decrease, reduce, attenuation, *etc.*

The weakening may also include a case where the activity of the polypeptide itself is decreased or eliminated due to variation of the polynucleotide encoding the polypeptide, *etc.,* as compared to the activity of the polypeptide originally possessed by the microorganism, a case where the overall polypeptide activity level and/or concentration (expression level) in the cell is low due to inhibition of the expression of the gene of the polynucleotide encoding the polypeptide or by inhibition of translation into the polypeptide, as compared to that of the natural strain, a case where the polynucleotide is not expressed at all, and/or a case where the polypeptide activity is absent even when the polynucleotide is expressed. The "endogenous activity" means the activity of a specific polypeptide originally possessed by the parent strain before change of the trait or a wild-type or unmodified microorganism when the trait is changed by genetic variation due to natural or artificial factors. The endogenous activity may be used interchangeably with "activity before modification". The fact that the activity of a polypeptide is "inactivated, deficient, decreased, down-regulated, reduced, or attenuated" as compared to the endogenous activity means that the activity of a polypeptide is lowered, as compared to the activity of a specific polypeptide originally possessed by the parent strain before change of the trait or the unmodified microorganism.

Such weakening of the activity of a polypeptide may be performed by any method known in the art, but the method is not limited thereto, and the weakening may be achieved by applying various methods well known in the art (*e.g*., Nakashima N et al., Bacterial cellular engineering by genome editing and gene silencing. Int J Mol Sci. 2014;15(2):2773-2793; Sambrook et al. Molecular Cloning 2012; *etc.).*

Specifically, the weakening of the polypeptide activity of the present disclosure may be:
1) deletion of the entirety or a part of the gene encoding the polypeptide;
2) modification of an expression regulatory region (or expression regulatory sequence) to decrease expression of the gene encoding the polypeptide;
3) modification of an amino acid sequence constituting the polypeptide to eliminate or weaken the activity of the polypeptide (*e.g*., deletion/substitution/addition of one or more amino acids in the amino acid sequence);
4) modification of a gene sequence encoding the polypeptide to eliminate or weaken the activity of the polypeptide (*e.g*., deletion/substitution/addition of one or more nucleic acid bases in a nucleic acid base sequence of the polypeptide gene to encode the polypeptide that has been modified to eliminate or weaken the activity of the polypeptide);
5) modification of a start codon of a gene transcript encoding the polypeptide or a base sequence encoding a 5'-UTR region;
6) introduction of an antisense oligonucleotide (*e.g*., antisense RNA) that complementarily binds to the transcript of the gene encoding the polypeptide;
7) addition of a sequence complementary to a Shine-Dalgarno sequence in front of the Shine-Dalgarno sequence of the gene encoding the polypeptide in order to form a secondary structure to which ribosome cannot be attached;
8) addition of a promoter to be transcribed in the opposite direction to the 3' end of the open reading frame (ORF) of the gene sequence encoding the polypeptide (reverse transcription engineering, RTE); or
9) a combination of two or more selected from 1) to 8), but is not particularly limited thereto.

For example,
1) the deletion of a part or the entirety of the gene encoding the polypeptide may be removal of the entire polynucleotide encoding the intrinsic polypeptide of interest in the chromosome, replacement with a polynucleotide in which some nucleotides are deleted, or replacement with a marker gene.

Further, 2) the modification of the expression regulatory region (or expression regulatory sequence) may be occurrence of variation in the expression regulatory region (or expression regulatory sequence) due to deletion, insertion, non-conservative or conservative substitution, or a combination thereof, or replacement with a sequence exhibiting weaker activity. The expression regulatory region includes a promoter, an operator sequence, a sequence encoding a ribosome binding site, and a sequence regulating the termination of transcription and translation, but is not limited thereto.

Further, 5) the modification of a start codon of a gene transcript encoding the polypeptide or a base sequence encoding a 5'-UTR region may be, for example, substitution with a base sequence encoding another start codon having a lower polypeptide expression rate, as compared to an intrinsic start codon, but is not limited thereto.

Further, 3) and 4) the modification of the amino acid sequence or polynucleotide sequence may be occurrence of variation in the sequence due to deletion, insertion, or non-conservative or conservative substitution of the amino acid sequence of the polypeptide or the polynucleotide sequence encoding the polypeptide or a combination thereof, or replacement with an amino acid sequence or a polynucleotide sequence modified to exhibit weaker activity or an amino acid sequence or a polynucleotide sequence modified to be inactive so that the activity of the polypeptide is weakened, but is not limited thereto. For example, expression of the gene may be inhibited or weakened by introducing variation into the polynucleotide sequence and forming a stop codon, but is not limited thereto.

Further, 6) the introduction of an antisense oligonucleotide (*e.g*., antisense RNA) that complementarily binds to the transcript of the gene encoding the polypeptide, may refer to documents, for example, [Weintraub, H. et al., Antisense-RNA as a molecular tool for genetic analysis, Reviews - Trends in Genetics, Vol. 1(1) 1986].

Further, 7) the addition of a sequence complementary to a Shine-Dalgarno sequence in front of the Shine-Dalgarno sequence of the gene encoding the polypeptide in order to form a secondary structure to which ribosome cannot be attached may be to make mRNA translation impossible or to slow down the mRNA translation rate.

Further, 8) the addition of a promoter to be transcribed in the opposite direction to the 3' end of the open reading frame (ORF) of the gene sequence encoding the polypeptide (reverse transcription engineering, RTE) may be to weaken the activity by making an antisense nucleotide complementary to the transcript of the gene encoding the polypeptide.

As used herein, the term "enhancement" of polypeptide activity means that the activity of a polypeptide is increased as compared to the intrinsic activity. The enhancement may be used interchangeably with terms such as activation, up-regulation, overexpression, increase, *etc.* Here, activation, enhancement, up-regulation, overexpression, and increase may include both exhibiting activity that was not originally possessed and exhibiting improved activity, as compared to the intrinsic activity or activity before modification. The "intrinsic activity" means activity of a specific polypeptide originally possessed by a parent strain before change of the trait or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased", as compared to the intrinsic activity, means that the activity of a polypeptide is improved, as compared to the activity and/or concentration (expression level) of a specific polypeptide originally possessed by a parent strain before change of the trait or an unmodified microorganism.

The enhancement may be achieved through the introduction of a foreign polypeptide or the enhancement of intrinsic activity and/or concentration (expression level) of the polypeptide. The activity enhancement of a polypeptide may be confirmed by an increase in the degree of activity and the expression level of the corresponding polypeptide or in the amount of a product produced from the corresponding polypeptide.

For the activity enhancement of the polypeptide, various methods well known in the art may be applied, and the method is not limited as long as the activity of the polypeptide of interest may be enhanced, as compared to that of the microorganism before being modified. Specifically, genetic engineering and/or protein engineering well known to those skilled in the art, which are routine methods of molecular biology, may be used, but the method is not limited thereto (*e.g*., Sitnicka et al. Functional Analysis of Genes. Advances in Cell Biology. 2010, Vol. 2. 1-16; Sambrook et al. Molecular Cloning 2012; *etc.).*

Specifically, the enhancement of the polypeptide activity of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a start codon of a gene transcript encoding the polypeptide or a base sequence encoding a 5'-UTR region;
4) modification of the amino acid sequence of the polypeptide to enhance the activity of the polypeptide;
5) modification of the polynucleotide sequence encoding the polypeptide to enhance the activity of the polypeptide (*e.g*., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the polypeptide;
7) codon optimization of a polynucleotide encoding the polypeptide;
8) analysis of the tertiary structure of the polypeptide to select the exposed site and to perform modification or chemical modification of the exposed site; or
9) a combination of two or more selected from 1) to 8), but is not particularly limited thereto.

More specifically, 1) the increase in the intracellular copy number of the polynucleotide encoding the polypeptide may be performed by introducing a vector, which replicates and functions irrespective of a host cell and is operably linked to the polynucleotide encoding the corresponding polypeptide, into a host cell. Alternatively, the increase may be achieved by the introduction of one copy or two or more copies of the polynucleotide encoding the corresponding polypeptide into a chromosome of a host cell. The introduction into the chromosome may be performed by introducing a vector, which is able to insert the polynucleotide into a chromosome of a host cell, into the host cell, but is not limited thereto. The vector is as described above.

2) The replacement of a gene expression control region (or expression control sequence) on a chromosome encoding a polypeptide with a sequence exhibiting strong activity may be, for example, occurrence of variation in a sequence due to deletion, insertion, non-conservative or conservative substitution, a combination thereof, or replacement with a sequence exhibiting stronger activity so that the activity of the expression control region is further enhanced. The expression control region is not particularly limited thereto, but may include a promoter, an operator sequence, a sequence encoding a ribosome binding site, a sequence controlling the termination of transcription and translation, and the like. For example, the replacement may be to replace the original promoter with a strong promoter, but is not limited thereto.

Examples of known strong promoters include CJ1 to CJ7 promoters (U.S. Patent No. 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent No. 10584338 B2), O2 promoter (U.S. Patent No. 10273491 B2), tkt promoter, yccA promoter, *etc.,* but are not limited thereto.

3) The modification of a start codon of the gene transcript encoding the polypeptide or a base sequence encoding a 5'-UTR region may be, for example, substitution with a base sequence encoding another start codon having a higher polypeptide expression rate, as compared to an endogenous start codon, but is not limited thereto.

4) and 5) The modification of the amino acid sequence or polynucleotide sequence may be occurrence of variation in the sequence due to deletion, insertion, non-conservative or conservative substitution of an amino acid sequence of the polypeptide or a polynucleotide sequence encoding the polypeptide, or a combination thereof, or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion. The selection marker is as described above.

6) The introduction of a foreign polynucleotide exhibiting the activity of the polypeptide may be the introduction of a foreign polynucleotide encoding a polypeptide exhibiting activity the same as or similar to that of the polypeptide into a host cell. The foreign polynucleotide is not limited in its origin or sequence as long as it exhibits activity the same as or similar to that of the polypeptide. The method used in the introduction may be performed by appropriately selecting a known transformation method by those skilled in the art. As the introduced polynucleotide is expressed in a host cell, a polypeptide may be produced, and the activity thereof may be increased.

7) The codon optimization of the polynucleotide encoding the polypeptide may be codon optimization of an endogenous polynucleotide so as to increase transcription or translation in a host cell or codon optimization of a foreign polynucleotide so as to perform optimized transcription and translation in a host cell.

8) The analysis of the tertiary structure of the polypeptide to select the exposed site and to perform modification or chemical modification of the exposed site may be, for example, to determine a template protein candidate according to the degree of similarity of the sequence by comparing the sequence information of a polypeptide to be analyzed with a database storing the sequence information of known proteins, to confirm the structure based on this, and to select and to modify or chemically modify the exposed portion to be modified or chemically modified.

Such enhancement of the polypeptide activity may be an increase in the activity or concentration/expression level of the corresponding polypeptide, based on the activity or concentration of the polypeptide expressed in a wild-type or a microbial strain before being modified, or an increase in the amount of a product produced from the corresponding polypeptide, but is not limited thereto.

As used herein, the term "regulation" of the polypeptide activity may be "enhancement" of the polypeptide activity and/or "weakening" of the polypeptide activity, but is not limited thereto. In one embodiment, the activity of GltA may be enhanced or weakened, but is not limited thereto.

Still another aspect of the present disclosure provides a method of producing poly-4-hydroxybutyrate (poly(4-hydroxybutyrate); P4HB), the method including the culturing the microorganism of the present disclosure.

As used herein, the term "poly-4-hydroxybutyrate", which is a polymer of 4-hydroxybutyrate, is a compound belonging to polyester. The poly-4-hydroxybutyrate may be used interchangeably with poly-4-hydroxybutanoate (P4HA), and may be represented by the following Chemical Formula 1, but is not limited thereto. 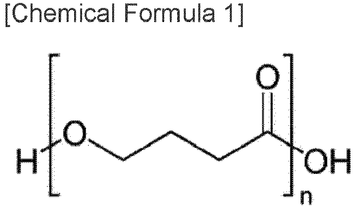 (wherein n is an integer of 1 or more)

As used herein, the term "culturing" means growing the microorganism of the present disclosure under appropriately controlled environmental conditions. In the present disclosure, the culturing process may be performed according to suitable medium and culture conditions known in the art. Such a culturing process may be easily adjusted and used by those skilled in the art according to the selected strain. Specifically, the culturing may be a batch type, continuous type, and/or fed-batch type, but is not limited thereto.

The microorganism of the present disclosure may be cultured in a common medium containing proper carbon sources, nitrogen sources, phosphorus sources, inorganic compounds, amino acids and/or vitamins, *etc.,* while controlling the temperature, pH, *etc.* under aerobic conditions.

In the culturing of the present disclosure, the culture temperature may be maintained at 20°C to 35°C, specifically, at 25°C to 35°C, at 28°C to 35°C, and the microorganism may be cultured for about 10 hours to about 160 hours, about 20 hours to about 130 hours, about 24 hours to about 120 hours, about 36 hours to about 120 hours, about 48 hours to about 120 hours, about 48 hours, about 72 hours, or about 120 hours, but are not limited thereto.

Poly-4-hydroxybutyrate produced through the culturing of the present disclosure may be secreted into the medium or may remain in the microorganisms.

The method of producing poly-4-hydroxybutyrate of the present disclosure may further include the preparing the microorganism of the present disclosure, the preparing a medium for culturing the microorganism, or a combination thereof (in any order), for example, prior to the culturing.

The method of producing poly-4-hydroxybutyrate of the present disclosure may further include the recovering poly-4-hydroxybutyrate from the medium according to culturing of the microorganism (the medium subjected to culturing) or from the microorganism of the present disclosure. The recovering may be further included after the culturing.

The recovering may be to collect poly-4-hydroxybutyrate of interest by way of a suitable method known in the art according to the method of culturing the microorganism of the present disclosure, for example, a batch, continuous, or fed-batch culture method. For example, centrifugation, filtration, treatment with a crystallized protein precipitant (salting out), extraction, cell lysis, ultrasonic disintegration, ultrafiltration, dialysis, various forms of chromatography such as molecular sieve chromatography (gel filtration), adsorption chromatography, ionexchange chromatography, and affinity chromatography, HPLC, or a combination thereof may be used. The poly-4-hydroxybutyrate of interest may be recovered from the medium or microorganism by way of a suitable method known in the art.

Further, the method of producing poly-4-hydroxybutyrate of the present disclosure may further include a purification step. The purification may be performed by way of a suitable method known in the art. For example, when the method of producing poly-4-hydroxybutyrate of the present disclosure includes both the recovering step and the purification step, the recovery step and the purification step may be performed discontinuously (or continuously) regardless of the order, or may be performed simultaneously or by being combined into one step, but is not limited thereto.

Still another aspect of the present disclosure provides a method of producing 1,4-butanediol, the method including the culturing the microorganism of the present disclosure; the recovering poly-4-hydroxybutyrate from the microorganism or the medium; and the degrading poly-4-hydroxybutyrate to 1,4-butanediol.

The method of producing 1,4-butanediol of the present disclosure may further include the degrading poly-4-hydroxybutyrate, which is produced by the microorganism of the present disclosure, to 1,4-butanediol. In the method of producing 1,4-butanediol of the present disclosure, the degrading may be further included after the culturing or the recovering. The degrading may be performed by way of a suitable method known in the art. In one embodiment, the degrading poly-4-hydroxybutyrate to 1,4-butanediol may be performed by pyrolysis, hydrogenation, or a combination thereof.

Still another aspect of the present disclosure provides a composition for producing poly-4-hydroxybutyrate, the composition including the microorganism of the present disclosure or a culture thereof.

The microorganism, poly-4-hydroxybutyrate, *etc.* is as described in other aspects.

The composition of the present disclosure may further include arbitrary suitable excipients commonly used. Such excipients may be, for example, a preservative, a wetting agent, a dispersing agent, a suspending agent, a buffering agent, a stabilizer, or an isotonic agent, but are not limited thereto.

Still another aspect of the present disclosure provides use of the microorganism of the present disclosure or a culture thereof in the production of poly-4-hydroxybutyrate.

Still another aspect of the present disclosure provides use of the microorganism of the present disclosure or a culture thereof in the production of 1,4-butanediol.

The microorganism, poly-4-hydroxybutyrate, *etc.* is as described in other aspects.

### [Mode for Carrying Out the Invention]

Hereinafter, the present disclosure will be described in more detail with reference to Examples. However, the following Examples are only preferred exemplary embodiments for illustrating the present disclosure, and therefore, it is not intended to limit the scope of the present disclosure thereto. Meanwhile, contents that are not described in the present specification may be sufficiently understood and easily implemented by those skilled in the art or similar art.

### Example 1: Regulation of phosphoenolpyruvate carboxylase expression and P4HB production using oxidative TCA cycle

### 1-1: P4HB production pathway using oxidative TCA (oTCA) cycle

P4HB production was evaluated in *E. coli* using glucose. Glucose may be oxidized to one phosphoenolpyruvate (PEP) and one acetyl-CoA and carbon dioxide through a phosphotransferase system. PEP fixes one carbon dioxide and may be converted to oxaloacetate by phosphoenolpyruvate carboxylase (PEP carboxylase; PPC). Acetyl-CoA and oxaloacetate may be oxidized via citrate to two carbon dioxides and one succinyl-CoA. Poly-4-hydroxybutyrate (P4HB) may be produced from succinyl-CoA which is an intermediate in the TCA cycle. The production of P4HB from succinyl-CoA is converted to succinyl semialdehyde by NADH or NADPH-dependent succinate semialdehyde dehydrogenase (SucD). Succinyl semialdehyde is converted to 4-hydroxybutyrate (4HB) by NADH-dependent 4-hydroxybutyric acid dehydrogenase (4HbD), which in turn may be converted to 4-hydroxybutyryl-CoA by 4-hydroxybutyryl-CoA transferase (OrfZ). Finally, poly-4-hydroxybutyrate (P4HB) was produced from 4-hydroxybutyryl-CoA by poly(3-hydroxyalkanoate) polymerase (PhaC).

The above-described SucD, 4HbD, OrfZ, PhaC, PPC, and gene sequences thereof are shown in SEQ ID NO: 1 to SEQ ID NO: 8, SEQ ID NO: 13, and SEQ ID NO: 14.

### 1-2: Construction of promoter for regulating phosphoenolpyruvate carboxylase expression

By limiting a nitrogen source essential for cell growth, it was intended to increase P4HB productivity by suppressing cell growth and by inducing carbon flux to the target material, P4HB. However, since expression of a promoter of the ppc gene encoding *E*. *coli's* phosphoenolpyruvate carboxylase (PEP carboxylase) is inhibited by a regulator (Nac, DNA-binding transcriptional dual regulator) under nitrogen-limiting conditions, it was tried to construct a promoter to overcome this problem.

To this end, a promoter which has an expression intensity equal to or higher than that of the wild promoter (Pn) and is not affected by the regulator Nac was constructed. The Pn promoter sequence is as follows.

5′-
TCGCAGCATTTGACGTCACCGCTTTTACGTGGCTTTATAAAAGACGACGAAAAG
CAAAGCCCGAGCATATTCGCGCCAATGCGACGTGAAGGATACAGGGCTATCAA
ACGATAAGATGGGGTGTCTGGGGTAAT-3′ (SEQ ID NO: 60)

As a result, as shown in Table 1 below, one promoter (Psynk1), which has the same strength as Pn, and one promoter (PsynK2), which is about 15 times stronger than Pn, were identified.

**[Table 1]**

| Type of promoter | GFP/OD |
|---|---|
| Pn | 2.2 ± 0.3 |
| PsynK1 | 2.6 ± 0.4 |
| PsynK2 | 32.2 ± 2.2 |

An M9 minimal medium containing glucose as a carbon source was used as a medium. After incubation in a 96-well plate under conditions of 48 hours, 800 rpm, and 37°C, absorbance was measured.

### 1-3: Preparation of P4HB-producing strain

The three promoters constructed in Example 1-2 were introduced into a microbial strain having the P4HB biosynthetic pathway of Example 1-1, respectively and the following three microorganisms, which are P4HB-producing strains, were prepared. The following SucD, 4HbD, OrfZ, PhaC, PPC, and their gene sequences are shown in SEQ ID NO: 1 to SEQ ID NO: 8, SEQ ID NO: 13, and SEQ ID NO: 14.

**[Table 2]**

| Strain | Representative genotype |
|---|---|
| #1 | Pn-ppc, PuspA-sucD*-4hbD*-phaC* |
| #2 | PsynK2-ppc, PuspA-sucD*-4hbD*-phaC* |
| #3 | PsynK1-ppc, PuspA-sucD*-4hbD*-phaC* |

The promoters used for the expression of the target gene are PuspA, PsynK1, and PsynK2, and gene information thereof may be obtained from ecocyc.org and parts.igem.org. The sequences of the promoters used are as follows.
- PsynK1 (5'-tttacagctagctcagtcctaggtattatgctagc-3'); SEQ ID NO: 45
- PsynK2 (5'-ctgacagctagctcagtcctaggtataatgctagc-3'); SEQ ID NO: 46

- PuspA (5'-
AACCACTATCAATATATTCATGTCGAAAATTTGTTTATCTAACGAGTAAGCAAGG
CGGATTGACGGATCATCCGGGTCGCTATAAGGTATAGTTCGCAGGACGCGGGT
GACGTAACGGCACAAGAAACG-3'); SEQ ID NO: 47

A commonly used technology was applied to the preparation of microorganisms in which the above-described P4HB biosynthetic pathway was established (representative literature: Sambrook et al., Molecular Cloning: A Laboratory Manual, Third Ed., Cold Spring Harbor Laboratory, New York (2001)). In detail, the target gene was introduced using ligase into a desired vector (pCL) by obtaining gene information from a known database, preparing the gene and primers therefor, and amplifying the target gene through polymerase chain reaction (PCR). Alternatively, a chemical synthesis method was also used, which was used when codon optimization was required to promote the expression in *E. coli.* For the expression of the target gene, the promoter and terminator were linked to the target gene through PCR. In order to introduce the vector thus prepared into the target strain, a general heat shock technique was used. An electroporation technique was used when higher efficiency was needed.

In addition to gene expression, attenuation/deletion of specific genes on the chromosome were performed using oligomers, and generally known Red/ET recombineering was used, and related techniques were referred to those reported in Datsenko and Wanner (Proc. Natl. Acad. Sci. USA, 2000, 97, 6640-6645). The oligomers used for deletion are as follows.
pykF-Left (SEQ ID NO: 48)
   GAAAGCAAGTTTCTCCCATCCTTCTCAACTTAAAGACTAAGACTGTCATG
pykF-right (SEQ ID NO: 49)
   GATATACAAATTAATTCACAAAAGCAATATTACAGGACGTGAACAGATGC
pykA-Left (SEQ ID NO: 50)
   TTTCATGTTCAAGCAACACCTGGTTGTTTCAGTCAACGGAGTATTACATG
pykA-right (SEQ ID NO: 51)
   TGGCGTTTTCGCCGCATCCGGCAACGTACTTACTCTACCGTTAAAATACG
maeB-left (SEQ ID NO: 52) 
TTCAGGGTAAGCGTGAGAGTTAAAAAAAATTACAGCGGTTGGGTTTGCG
C
maeB-right (SEQ ID NO: 53)
   TTGCCCACACACTTTATTTGTGAACGTTACGTGAAAGGAACAACCAAATG
maeA-left (SEQ ID NO: 54) 
CCCGGTAGCCTTCACTACCGGGCGCAGGCTTAGATGGAGGTACGGCGG
TA
maeA-right (SEQ ID NO: 55) 
GGCCGACGCCCTGGCGGTAAAGCAAAGACGATAAAAGCCCCCCAGGGA
TG
aspC-left (SEQ ID NO: 56)
   TTTTCAGCGGGCTTCATTGTTTTTAATGCTTACAGCACTGCCACAATCGC
aspC-right (SEQ ID NO: 57)
   TACCCTGATAGCGGACTTCCCTTCTGTAACCATAATGGAACCTCGTCATG
Icd-left (SEQ ID NO: 58) 
AACGTGGTGGCAGACGAGCAAACCAGTAGCGCTCGAAGGAGAGGTGAA
TG
Icd-right (SEQ ID NO: 59)
   CCCGTTAATAAATTTAACAAACTACGGCATTACATGTTTTCGATGATCGC

### 1-4: Evaluation of P4HB productivity of P4HB-producing strain

A flask test was performed to test the P4HB production of the three strains prepared in Example 1-3. The culture conditions were 48 hours, 230 rpm, and 30°C, and a medium was prepared, based on previously published data, and used (U.S. Patent No. 9,084,467 B2). In detail, a medium was prepared by adding 1× trace salt solution to 1×E2 minimal medium, and used after adjusting its carbon:nitrogen ratio (C/N ratio) to 30:1.

P4HB analysis conditions were set by referring to a published literature (U.S. Patent No. 9,084,467 B2). Briefly, each 1 mL of the cultures of the three strains as described above was collected and the cells were recovered at 4,000 rpm. After lyophilizing each of the recovered strains, a preparation for butanolysis of the sample (a preparation obtained by adding 99.9% butanol and 4 N HCl to a dioxane solution) was added and heat-treated at 93°C for 6 hours. The heat-treated solution was phase-separated at 600 rpm, and an organic phase was collected and analyzed by gas chromatography (GC). A standard reagent of 4-hydroxybutyrate (4HB) was prepared using 10% γ-butyrolactone.

As a result of the analysis using the above conditions, as shown in Table 3 below, the P4HB concentration was confirmed to be improved by 22% in the strain with PsynK1, which is a promoter without nitrogen limitation, as compared to the strain with Pn.

**[Table 3]**

| Strain | P4HB concentration (g/L) |
|---|---|
| #1 | 14.13 ± 0.05 |
| #2 | 14.81 ± 0.10 |
| #3 | 17.29 ± 0.02 |

On the other hand, it was further confirmed from Table 3 that when an excessively strong promoter (PsynK2) was used, P4HB productivity was not improved even though it was not affected by nitrogen-limiting factors.

### Example 2: P4HB production using reductive TCA (rTCA) cycle

Carbon dioxide fixed by the carboxylation reaction of phosphoenolpyruvate carboxylase was induced into the P4HB production pathway using a reductive TCA cycle. In the production of P4HB through the reductive TCA cycle, oxaloacetate produced from phosphoenolpyruvate through the phosphoenolpyruvate carboxylase enzyme does not undergo a decarboxylation process included in an oxidative TCA cycle, and thus it may be reduced to succinyl-CoA through malate, fumarate, and succinate without generating carbon dioxide. The produced succinyl-CoA may be converted to P4HB through the same method as in the pathway of Example 1-1.

For efficient activation of the rTCA cycle, a strain (Strain No. 4 below) in which the pyruvate kinase gene (pykFA) was removed was prepared. The three strains prepared are shown in Table 4 below, and the following Strain No. 3 is the same as Strain No. 3 in Table 2 above. The following SucD, 4HbD, OrfZ, PhaC, PPC, and gene sequences thereof are shown in SEQ ID NO: 1 to SEQ ID NO: 8, SEQ ID NO: 13, and SEQ ID NO: 14. Further, the following MaeAB, AspC, PykFA, and gene sequences thereof are shown in SEQ ID NO: 9 to SEQ ID NO: 12, SEQ ID NO: 21 to SEQ ID NO: 24, SEQ ID NO: 29, and SEQ ID NO: 30.

**[Table 4]**

| Strain | Representative genotype |
|---|---|
| #3 | PsynK1-ppc, PuspA-sucD*-4hbD*-phaC* |
| #4 | ΔpykFA PsynK1-ppc, PuspA-sucD*-4hbD*-phaC* |
| #5 | ΔmaeAB ΔaspC ΔpykFA PsynK1-ppc, PupA-sucD*-4hbD*-phaC* |

When pyruvate kinase is removed, the carbon flux from PEP to pyruvate is controlled to preserve the intracellular concentration of PEP, and based on this, activation of the PEP carboxylase pathway may be promoted. This suggests that the intracellular concentration of oxaloacetate, which is essential for the rTCA cycle, may be increased.

Additionally, in order to eliminate the competitive pathways on the rTCA cycle, such as oxaloacetate and malate, *etc.,* a strain, in which aminotransferase (aspartate aminotransferase; AspC) and malate dehydrogenase (malic enzyme; MaeAB) were additionally removed, was also prepared (Strain No. 5 above) to perform rTCA-based P4HB production evaluation.

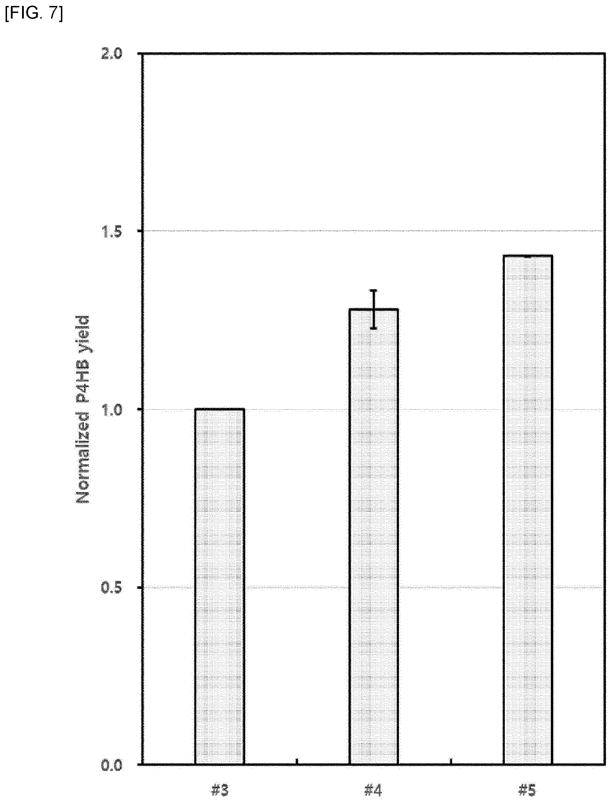

For the strains in Table 4, P4HB productivity was evaluated in the same manner as in Example 1-4, and when the rTCA cycle was activated, cell growth was improved, and as shown in FIG. 7, in the strains into which the rTCA cycle was introduced (Strain Nos. 4 and 5), the P4HB yield was confirmed to be improved from about 28% up to 43%, as compared to the strain (Strain No. 3) into which the rTCA cycle was not introduced. When the rTCA cycle is used, the theoretical maximum yield of P4HB may be improved from the existing 48 wt% to 58 wt%.

### Example 3: P4HB production using glyoxylate cycle

By producing P4HB using the glyoxylate cycle, the decarboxylation pathway of 2-oxoglutarate was bypassed to reduce loss of carbon to carbon dioxide, and consequently, to increase the production yield of P4HB.

For activation of this cycle, isocitrate dehydrogenase (icd) on the TCA cycle was removed to forcibly induce carbon flux into the glyoxylate cycle. For the activation of the glyoxylate cycle, E. coli-derived isocitrate lyase (aceA) and malate synthase (aceB) were introduced, but the existing wild-type promoter in the introduced microorganism was replaced with a synthetic promoter such that it was not subjected to glucose-based catabolic repression. The two strains prepared are shown in Table 5 below. The following SucD, 4HbD, OrfZ, PhaC, PPC, and gene sequences thereof are shown in SEQ ID NO: 1 to SEQ ID NO: 8, SEQ ID NO: 13, and SEQ ID NO: 14. Further, the following Icd, AceBA, and gene sequences thereof are shown in SEQ ID NO: 35, SEQ ID NO: 36, SEQ ID NO: 37, SEQ ID NO: 38, SEQ ID NO: 43, and SEQ ID NO: 44.

**[Table 5]**

| Strain | Representative genotype |
|---|---|
| #6 | Δicd PsynK1-ppc, PuspA-sucD*-4hbD*-phaC* |
| #7 | Δicd PsynK1-ppc, PuspA-sucD*-4hbD*-phaC*, pCL-PsynK1-aceBA |

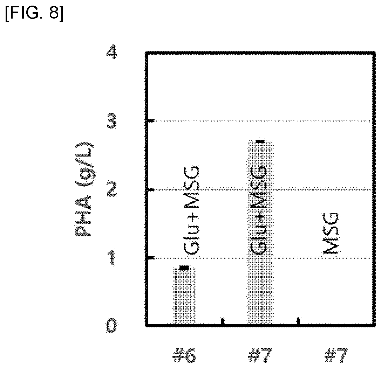

At this time, both succinate and malate, which are products of the glyoxylate cycle, may be converted to succinyl-CoA using the reductive TCA cycle. The produced succinyl-CoA may be converted to P4HB through the P4HB production pathway in Example 1-1. During fermentation of the strain thus prepared, 50 mM of monosodium glutamate (MSG) was added to externally supplement glutamate. As a result of examining PHA production by culturing the strain thus prepared in a glucose medium, as shown in FIG. 8, it was confirmed that 2.7 g/L of PHA was produced (Strain No. 7 GLU+MSG). In the strain into which the glyoxylate cycle was not introduced (Strain No. 6), PHA production was only 0.9 g/L, and even though the glyoxylate cycle was introduced, no PHA production was observed in the absence of glucose (Strain No. 7 MSG). Through this method, it was possible to improve from the existing theoretical maximum yield of 48 wt% to 58 wt%.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of producing 1,4-butanediol, the method comprising:
(1) converting succinyl-CoA (SuCoA) to succinate semialdehyde (SSA);
(2) converting succinate semialdehyde (SSA) to 4-hydroxybutyrate (4HB);
(3) converting 4-hydroxybutyrate (4HB) to 4-hydroxybutyryl CoA (4HBCoA);
(4) producing poly-4-hydroxybutyrate (P4HB) by polymerizing two or more of 4-hydroxybutyryl CoA (4HBCoA); and
(5) degrading poly-4-hydroxybutyrate to 1,4-butanediol.

2. The method of claim 1, wherein (1) to (4) use any one or more selected from the group consisting of any one or more polypeptides selected from the group consisting of succinate semialdehyde dehydrogenase, 4-hydroxybutyric acid dehydrogenase, 4-hydroxybutyryl-CoA transferase, and poly(3-hydroxyalkanoate) polymerase; a microorganism including the polypeptide, a polynucleotide encoding the polypeptide, or a combination thereof; and a culture thereof.

3. The method of claim 1, further comprising a TCA cycle.

4. The method of claim 3, wherein the TCA cycle includes any one or more selected from the group consisting of
(a1) converting pyruvate to acetyl-CoA;
(b1) converting acetyl-CoA and oxaloacetate to citrate;
(c1) converting citrate to isocitrate;
(d1) converting isocitrate to α-ketoglutarate;
(e1) converting α-ketoglutarate to succinyl-CoA; and
(f1) converting pyruvate to oxaloacetate.

5. The method of claim 1, further comprising (g1) converting phosphoenolpyruvate to oxaloacetate.

6. The method of claim 5, wherein (g1) uses any one or more selected from the group consisting of a phosphoenolpyruvate carboxylase polypeptide; a microorganism including the polypeptide, a polynucleotide encoding the polypeptide, or a combination thereof; and a culture thereof.

7. The method of claim 1, wherein (g1) the converting phosphoenolpyruvate to oxaloacetate is enhanced.

8. The method of claim 1, further comprising a reductive TCA cycle.

9. The method of claim 8, wherein the reductive TCA cycle includes any one or more selected from the group consisting of
(a2) converting oxaloacetate to malate;
(b2) converting malate to fumarate;
(c2) converting fumarate to succinate; and
(d2) converting succinate to succinyl-CoA.

10. The method of claim 8, wherein (e2) the converting phosphoenolpyruvate to pyruvate is weakened.

11. The method of claim 8, wherein the reductive TCA cycle is enhanced by any one or more selected from the group consisting of the following (I) to (XII):
(I) weakening of pyruvate kinase;
(II) enhancement of phosphoenolpyruvate carboxylase (PEP carboxylase);
(III) enhancement of carbonic anhydrase;
(IV) regulation of citrate synthase;
(V) enhancement of pyruvate carboxylase;
(VI) weakening of NAD⁺-dependent malate dehydrogenase;
(VII) weakening of NADP⁺-dependent malate dehydrogenase;
(VIII) weakening of phosphogluconate dehydratase;
(IX) weakening of 2-keto-4-hydroxyglutarate:2-keto-3-deoxygluconate 6-phosphate aldolase (KHG/KDPG aldolase);
(X) weakening of aspartate aminotransferase;
(XI) weakening of glucose-specific PTS enzyme IIBC component; and
(XII) enhancement of bicarbonate transporter.

12. The method of claim 1, further comprising a glyoxylate cycle.

13. The method of claim 12, wherein the glyoxylate cycle further includes any one or more selected from the group consisting of
(a3) converting isocitrate to glyoxylate and succinate;
(b3) converting glyoxylate and acetyl-CoA to malate and CoA;
(c3) converting citrate to isocitrate;
(d3) converting pyruvate to oxaloacetate;
(e3) converting phosphoenolpyruvate to oxaloacetate;
(f3) converting oxaloacetate to citrate;
(g3) converting malate to fumarate;
(h3) converting fumarate to succinate; and
(i3) converting succinate to succinyl-CoA.

14. The method of claim 13, wherein (j3) converting α-ketoglutarate to succinyl-CoA is weakened.

15. The method of claim 13, wherein (k3) converting oxaloacetate to malate is weakened.

16. The method of claim 13, wherein the glyoxylate cycle is enhanced by any one or more selected from the group consisting of (i) to (vi):
(i) enhancement of citrate synthase;
(ii) weakening of isocitrate dehydrogenase;
(iii) enhancement of isocitrate lyase;
(iv) enhancement of isocitrate dehydrogenase kinase/phosphatase;
(v) enhancement of malate synthase G; and
(vi) enhancement of malate synthase A.

17. A microorganism comprising succinate semialdehyde dehydrogenase, 4-hydroxybutyric acid dehydrogenase, 4-hydroxybutyryl-CoA transferase, and poly(3-hydroxyalkanoate) polymerase polypeptide, a polynucleotide encoding the polypeptide, or a combination thereof.

18. The microorganism of claim 17, wherein any one or more polypeptides selected from the group consisting of succinate semialdehyde dehydrogenase, 4-hydroxybutyric acid dehydrogenase, 4-hydroxybutyryl-CoA transferase, and poly(3-hydroxyalkanoate) polymerase are introduced from foreign sources.

19. The microorganism of claim 17, wherein the succinate semialdehyde dehydrogenase and 4-hydroxybutyryl-CoA transferase polypeptide are derived from *Clostridium kluyveri*, the 4-hydroxybutyric acid dehydrogenase polypeptide is derived from *Arabidopsis thaliana*, and the poly(3-hydroxyalkanoate) polymerase is derived from *Pseudomonas putida* or *Ralstonia eutropha.*

20. The microorganism of claim 17, wherein the microorganism includes a TCA cycle.

21. The microorganism of claim 17, wherein the microorganism includes any one or more polypeptides selected from the group consisting of pyruvate dehydrogenase, citrate synthase, aconitase, isocitrate dehydrogenase, α-ketoglutarate dehydrogenase, and pyruvate carboxylase, a polynucleotide encoding the polypeptide, or a combination thereof.

22. The microorganism of claim 17, wherein the microorganism includes a reductive TCA cycle.

23. The microorganism of claim 17, wherein the microorganism includes any one or more selected from the group consisting of the following (I) to (XII):
(I) weakening of pyruvate kinase;
(II) enhancement of phosphoenolpyruvate carboxylase;
(III) enhancement of carbonic anhydrase;
(IV) regulation of citrate synthase;
(V) enhancement of pyruvate carboxylase;
(VI) weakening of NAD⁺-dependent malate dehydrogenase;
(VII) weakening of NADP⁺-dependent malate dehydrogenase;
(VIII) weakening of phosphogluconate dehydratase;
(IX) weakening of 2-keto-4-hydroxyglutarate:2-keto-3-deoxygluconate 6-phosphate aldolase (KHG/KDPG aldolase);
(X) weakening of aspartate aminotransferase;
(XI) weakening of glucose-specific PTS enzyme IIBC component; and
(XII) enhancement of bicarbonate transporter.

24. The microorganism of claim 17, wherein the microorganism includes a glyoxylate cycle.

25. The microorganism of claim 17, wherein the microorganism includes any one or more selected from the group consisting of the following (i) to (vi):
(i) enhancement of citrate synthase;
(ii) weakening of isocitrate dehydrogenase;
(iii) enhancement of isocitrate lyase;
(iv) enhancement of isocitrate dehydrogenase kinase/phosphatase;
(v) enhancement of malate synthase G; and
(vi) enhancement of malate synthase A.

26. The microorganism of claim 17, wherein the microorganism is used in the production of 1,4-butanediol.

27. The microorganism of claim 17, wherein the microorganism is for producing poly-4-hydroxybutyrate.

28. The microorganism of claim 17, wherein the microorganism belongs to the genus *Corynebacterium* or the genus *Escherichia.*

29. The microorganism of claim 17, wherein the microorganism has poly-4-hydroxybutyrate production ability even under limiting conditions of any one or more nutrients selected from the group consisting of nitrogen, sulfur, phosphorus, and magnesium.

30. The microorganism of claim 17, wherein the microorganism includes a nucleotide sequence having promoter activity, which is represented by SEQ ID NO: 45.

31. The microorganism of claim 30, wherein a target gene of the nucleotide sequence having promoter activity, which is represented by SEQ ID NO: 45, is a polynucleotide encoding phosphoenolpyruvate carboxylase.

32. A method of producing poly-4-hydroxybutyrate, the method comprising the culturing the microorganism of any one of claims 17 to 31.

33. The method of claim 32, further comprising the recovering poly-4-hydroxybutyrate from the microorganism or a medium.

34. The method of claim 32, wherein the culturing the microorganism includes the culturing the microorganism in a medium under limiting conditions of any one or more nutrients selected from the group consisting of nitrogen, sulfur, phosphorus, and magnesium.

35. A method of producing 1,4-butanediol, the method comprising the steps of:
culturing the microorganism of any one of claims 17 to 31;
recovering poly-4-hydroxybutyrate from the microorganism or a medium; and
degrading poly-4-hydroxybutyrate to 1,4-butanediol.

36. The method of claim 35, wherein the degrading poly-4-hydroxybutyrate to 1,4-butanediol is pyrolysis, hydrogenation, or a combination thereof.

37. A composition for producing poly-4-hydroxybutyrate, the composition comprising the microorganism of claim 17 or a culture thereof.

38. Use of the microorganism of claim 17 or a culture thereof in the production of poly-4-hydroxybutyrate.
